# EUROPEAN PATENT APPLICATION

(11) **EP 4 446 331 A1**
(43) Date of publication of application: **16.10.2024**
(21) Application number: 22904284.1
(22) Date of filing: 08.12.2022
(51) Int. Cl.: C07K 1/06, C07C 43/23, C07C 69/712, C07C 235/06, C07C 271/16

(54) **METHOD FOR PRODUCING PEPTIDE COMPOUND, REAGENT FOR FORMING PROTECTIVE GROUP, AND SUBSTITUTED BENZYL COMPOUND**

(30) Priority: 08.12.2021 JP 2021199281
(71) Applicant: FUJIFILM Corporation, Tokyo 106-8620 (JP)
(72) Inventor: NAKAGAWA, Daisuke, Ashigarakami-gun, Kanagawa 258-8577 (JP); TSUYAMA, Hiroaki, Ashigarakami-gun, Kanagawa 258-8577 (JP); YOSHIDA, Hayato, Ashigarakami-gun, Kanagawa 258-8577 (JP); MOGI, Takuma, Ashigarakami-gun, Kanagawa 258-8577 (JP)
(74) Representative: HGF
(86) International application number: PCT/JP2022/045206
(87) International publication number: WO 2023/106356

(57) **Abstract**

An object of the present invention is to provide a method for producing a peptide compound with an excellent yield, a protective group-forming reagent having an excellent yield, and a novel substituted benzyl compound. According to the present invention, there is provided a method for producing a peptide compound, including a step of using a substituted benzyl compound represented by Formula (1).

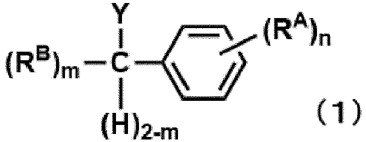

In Formula (1), Y represents -OH, -NHR, -SH, or -X, R represents a hydrogen atom, an alkyl group, an arylalkyl group, a heteroarylalkyl group, or a 9-fluorenylmethoxycarbonyl group, X represents Cl, Br, or I, m represents 1 or 2, n represents an integer of 1 to 5, R^{B}'s each independently represent an aliphatic hydrocarbon group, R^{A}'s each independently represent an aliphatic hydrocarbon group or an organic group having an aliphatic hydrocarbon group, where the number of carbon atoms in at least one aliphatic hydrocarbon group is 12 or more, and a benzene ring in Formula (1) may further have a substituent in addition to R^{A}.

## Description

### Technical Field

The present disclosure relates to a method for producing a peptide compound, a protective group-forming reagent, and a substituted benzyl compound.

### Background Art

Examples of a method for producing peptide include a solid phase method and a liquid phase method.

The solid phase method is advantageous in that isolation and purification after reaction can be performed by only washing resin. However, the solid phase method is associated with problems in that the reaction is essentially a heterogeneous phase reaction, a reaction agent needs to be used in excess to compensate for the low reactivity, and tracing of the reaction and analysis of a reaction product supported by a carrier are difficult.

On the other hand, the liquid phase method is advantageous in that good reactivity is exhibited, and intermediate peptide can be purified by extraction and washing, isolation, and the like after a condensation reaction. However, the liquid phase method still has problems in each step of coupling reaction and deprotection.

In addition, as a protective group-forming reagent in the related art, an alkoxy-substituted benzyl alcohol compound disclosed in Patent Document 1 is known.

### Prior Art Documents

### Patent Documents

Patent Document 1: WO2007/034812A

### SUMMARY OF THE INVENTION

### Object to be solved by the invention

An object to be achieved by an embodiment of the present invention is to provide a method for producing a peptide compound with an excellent yield.

An object to be achieved by another embodiment of the present invention is to provide a protective group-forming reagent with an excellent yield.

An object to be achieved by still another embodiment of the present invention is to provide a novel substituted benzyl compound.

### Means for solving the object

The methods for achieving the above-described objects include the following aspects.
<1> A method for producing a peptide compound, comprising:
   a step of using a substituted benzyl compound represented by Formula (1), 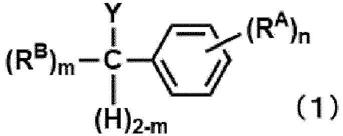
   in Formula (1), Y represents -OH, -NHR, -SH, or -X, R represents a hydrogen atom, an alkyl group, an arylalkyl group, a heteroarylalkyl group, or a 9-fluorenylmethoxycarbonyl group, X represents Cl, Br, or I, m represents 1 or 2, n represents an integer of 1 to 5,
   R^{B}'s each independently represent an aliphatic hydrocarbon group,
   R^{A}'s each independently represent an aliphatic hydrocarbon group or an organic group having an aliphatic hydrocarbon group, where a number of carbon atoms in at least one aliphatic hydrocarbon group is 12 or more, and a benzene ring in Formula (1) may further have a substituent in addition to R^{A}.
<2> The method for producing a peptide compound according to <1>,
   in which the step of using the substituted benzyl compound represented by Formula (1) is a C-terminal protecting step of protecting a carboxy group or an amide group of an amino acid compound or a peptide compound with the substituted benzyl compound represented by Formula (1).
<3> The method for producing a peptide compound according to <2>,
   in which the amino acid compound or the peptide compound in the C-terminal protecting step is an N-terminal protected amino acid compound or an N-terminal protected peptide compound.
<4> The method for producing a peptide compound according to <3>, further comprising:
   an N-terminal deprotecting step of deprotecting an N-terminal end of an N-terminal and C-terminal protected amino acid compound or an N-terminal and C-terminal protected peptide compound, which is obtained in the C-terminal protecting step; and
   a peptide chain extending step of condensing the N-terminal end of a C-terminal protected amino acid compound or a C-terminal protected peptide compound, which is obtained in the N-terminal deprotecting step, with an N-terminal protected amino acid compound or an N-terminal protected peptide compound.
<5> The method for producing a peptide compound according to <4>, further comprising:
   a precipitating step of precipitating an N-terminal and C-terminal protected peptide compound obtained in the peptide chain extending step.
<6> The method for producing a peptide compound according to <5>, further comprising, one or more times in the following order after the precipitating step:
   a step of deprotecting an N-terminal end of the obtained N-terminal and C-terminal protected peptide compound;
   a step of condensing the N-terminal end of the obtained C-terminal protected peptide compound with an N-terminal protected amino acid compound or an N-terminal protected peptide compound; and
   a step of precipitating the obtained N-terminal and C-terminal protected peptide compound.
<7> The method for producing a peptide compound according to any one of <1> to <6>, further comprising:
   a C-terminal deprotecting step of deprotecting a C-terminal protective group.
<8> The method for producing a peptide compound according to any one of <1> to <7>,
   in which a total number of carbon atoms in all aliphatic hydrocarbon groups included in all R^{A}'s is 36 to 80.
<9> A protective group-forming reagent comprising:
   a substituted benzyl compound represented by Formula (1), 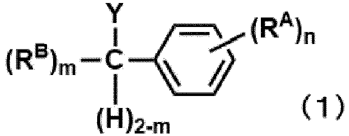
   in Formula (1), Y represents -OH, -NHR, -SH, or -X, R represents a hydrogen atom, an alkyl group, an arylalkyl group, a heteroarylalkyl group, or a 9-fluorenylmethoxycarbonyl group, X represents Cl, Br, or I, m represents 1 or 2, n represents an integer of 1 to 5,
   R^{B}'s each independently represent an aliphatic hydrocarbon group,
   R^{A}'s each independently represent an aliphatic hydrocarbon group or an organic group having an aliphatic hydrocarbon group, where a number of carbon atoms in at least one aliphatic hydrocarbon group is 12 or more, and a benzene ring in Formula (1) may further have a substituent in addition to R^{A}.
<10> The protective group-forming reagent according to <9>,
   in which the protective group-forming reagent is a reagent for forming a protective group of a carboxy group or an amide group.
<11> The protective group-forming reagent according to <9> or <10>,
   in which the protective group-forming reagent is a C-terminal protective group-forming reagent of an amino acid compound or a peptide compound.
<12> A substituted benzyl compound represented by Formula (1), 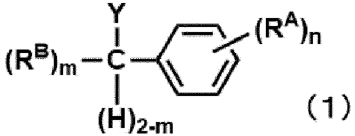
   in Formula (1), Y represents -OH, -NHR, -SH, or -X, R represents a hydrogen atom, an alkyl group, an arylalkyl group, a heteroarylalkyl group, or a 9-fluorenylmethoxycarbonyl group, X represents Cl, Br, or I, m represents 1 or 2, n represents an integer of 1 to 5,
   R^{B}'s each independently represent an aliphatic hydrocarbon group,
   R^{A}'s each independently represent an aliphatic hydrocarbon group or an organic group having an aliphatic hydrocarbon group, where a number of carbon atoms in at least one aliphatic hydrocarbon group is 12 or more, and a benzene ring in Formula (1) may further have a substituent in addition to R^{A}.
<13> A method for producing a peptide compound, comprising:
   a step of using a substituted benzyl compound represented by Formula (1), 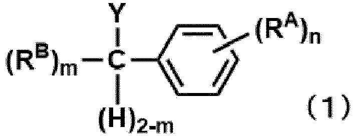
   in Formula (1), Y represents -OH, -NHR, -SH, or -X, R represents a hydrogen atom, an alkyl group, an arylalkyl group, a heteroarylalkyl group, or a 9-fluorenylmethoxycarbonyl group, X represents Cl, Br, or I, m represents 1 or 2, n represents an integer of 1 to 5,
   R^{B}'s each independently represent an aromatic group (excluding a phenyl group),
   R^{A}'s each independently represent an aliphatic hydrocarbon group or an organic group having an aliphatic hydrocarbon group, where a number of carbon atoms in at least one aliphatic hydrocarbon group is 12 or more, and a benzene ring in Formula (1) may further have a substituent in addition to R^{A}.
<14> The method for producing a peptide compound according to <13>,
   in which R^{B} represents a naphthalene group.

### Effect of the invention

According to an embodiment of the present invention, it is possible to provide a method for producing a peptide compound with an excellent yield.

In addition, according to another embodiment of the present invention, it is possible to provide a protective group-forming reagent with an excellent yield.

In addition, according to still another embodiment of the present invention, it is possible to provide a novel substituted benzyl compound.

### DESCRIPTION OF THE PREFERRED EMBODIMENTS

Hereinafter, the contents of the present disclosure will be described in detail. The description of constituent elements below is made based on representative embodiments of the present disclosure in some cases, but the present disclosure is not limited to such embodiments.

In the present specification, unless otherwise specified, each term has the following meaning.

A numerical range represented using "to" means a range including numerical values described before and after the preposition "to" as a lower limit value and an upper limit value.

In numerical ranges described in stages in the present specification, an upper limit value or a lower limit value described in one numerical range may be replaced with an upper limit value or a lower limit value of a numerical range described in another stage. In addition, in the numerical ranges described in the present specification, the upper limit value or the lower limit value of the numerical ranges may be replaced with the values shown in examples.

The term "step" includes not only the independent step but also a step in which intended purposes are achieved even in a case where the step cannot be precisely distinguished from other steps.

In a case where substitution or unsubstitution is not noted in regard to the notation of a "group" (atomic group), the "group" includes not only a group not having a substituent but also a group having a substituent. For example, the concept of an "alkyl group" includes not only an alkyl group not having a substituent (unsubstituted alkyl group) but also an alkyl group having a substituent (substituted alkyl group).

A chemical structural formula may be described by a simplified structural formula in which hydrogen atoms are omitted.
"% by mass" has the same definition as that for "% by weight", and "part by mass" has the same definition as that for "part by weight".

A combination of two or more preferred aspects is a more preferred aspect.

The alkyl group may be chain-like or branched, and may be substituted. Examples of the substituent include a halogen atom, a C₁₋₆ alkyl group, a C₃₋₈ cycloalkyl group, a C₂₋₆ alkenyl group, a C₂₋₆ alkynyl group, a C₁₋₆ acyl group, a C₁₋₆ alkoxy group, a C₆₋₂₀ aryl group, a C₅₋₂₀ heteroaryl group, and a heterocyclic group.

Unless otherwise specified, examples of the alkyl group include an alkyl group having 1 to 30 carbon atoms (also referred to as "number of carbon atoms"), and the alkyl group may be an alkyl group having 1 to 20 carbon atoms, an alkyl group having 1 to 10 carbon atoms, or an alkyl group having 1 to 6 carbon atoms. Examples of the alkyl group having 1 to 6 carbon atoms include methyl, ethyl, propyl, isopropyl, butyl, and tert-butyl.

The cycloalkyl group may be substituted with a halogen atom, an alkoxy group, or the like. The alkyl group may be a cycloalkyl group having 3 to 10 carbon atoms or an alkyl group having 3 to 6 carbon atoms.

Examples of the arylalkyl group include an arylalkyl group having 7 to 30 carbon atoms, and the arylalkyl group may be an arylalkyl group having 7 to 20 carbon atoms, an arylalkyl group having 7 to 16 carbon atoms (for example, a group in which an aryl group having 6 to 10 carbon atoms is bonded to an alkylene group having 1 to 6 carbon atoms), or an arylalkyl group having 7 to 10 carbon atoms. Specific examples thereof include a benzyl group, a 1-phenylethyl group, a 2-phenylethyl group, a 1-phenylpropyl group, a naphthylmethyl group, a 1-naphthylethyl group, and a 1-naphthylpropyl group, and a benzyl group is preferable.

The alkenyl group preferably has 2 to 30 carbon atoms, more preferably has 2 to 20 carbon atoms, still more preferably has 2 to 10 carbon atoms, and even more preferably has 2 to 6 carbon atoms. Examples of the alkenyl group include a pentenyl group, a hexenyl group, and an oleyl group.

The alkynyl group preferably has 2 to 30 carbon atoms, more preferably has 2 to 20 carbon atoms, still more preferably has 2 to 10 carbon atoms, and even more preferably has 2 to 6 carbon atoms. Examples of the alkynyl group include a 4-pentynyl group and a 5-hexenyl group.

Examples of the aliphatic hydrocarbon group include an alkyl group, a cycloalkyl group, an alkenyl group, and an alkynyl group. The aliphatic hydrocarbon group may be linear or branched, and may be substituted. The substituent is the same as the substituent of the alkyl group. The aliphatic hydrocarbon group preferably has 1 to 30 carbon atoms, more preferably has 1 to 20 carbon atoms, still more preferably has 1 to 10 carbon atoms, and even more preferably has 1 to 6 carbon atoms.

Examples of the aromatic group include an aryl group and a heteroaryl group.

The aryl group preferably has 6 to 30 carbon atoms, more preferably has 6 to 20 carbon atoms, still more preferably has 6 to 14 carbon atoms, and even more preferably has 6 to 10 carbon atoms. Specific examples thereof include a phenyl group, a 1-naphthyl group, a 2-naphthyl group, a biphenylyl group, and a 2-anthryl group. Among these, a phenyl group is preferable.

The number of constituent atoms in the heteroaryl group is preferably 5 to 30, more preferably 5 to 20, still more preferably 5 to 14, and even more preferably 5 to 10. As the heteroaryl group, a monocyclic or bicyclic heteroaryl group is preferable.

Examples of the monocyclic heteroaryl group include a monocyclic nitrogen-containing heteroaryl group, a monocyclic oxygen-containing heteroaryl group, a monocyclic sulfur-containing heteroaryl group, a monocyclic nitrogen and oxygen-containing heteroaryl group, and a monocyclic nitrogen and sulfur-containing heteroaryl group. Examples of the bicyclic heteroaryl group include a bicyclic heteroaryl group, a bicyclic oxygen-containing heteroaryl group, a bicyclic sulfur-containing heteroaryl group, a bicyclic nitrogen and oxygen-containing heteroaryl group, and a bicyclic nitrogen and sulfur-containing heteroaryl group. Suitable specific examples thereof include an indolyl group, a benzofuranyl group, a thiophenyl group, a benzothiophenyl group, a furyl group, a benzofuranylmethyl group, a thiophanylmethyl group, and a benzothiophanylmethyl group.

Examples of a silyl group include trimethylsilyl, triethylsilyl, dimethylphenylsilyl, tert-butyldimethylsilyl, and tert-butyldiethylsilyl.

Examples of a halogen atom include a fluorine atom, a chlorine atom, a bromine atom, and an iodine atom.

Examples of an alkoxy group having 1 to 6 carbon atoms include methoxy, ethoxy, and propoxy.

Examples of an acyl group having 1 to 6 carbon atoms include acetyl and propionyl.

Examples of an arylalkylcarbonyl group having 7 to 10 carbon atoms include benzylcarbonyl.

Examples of an alkoxycarbonyl group having 2 to 6 carbon atoms include methoxycarbonyl, ethoxycarbonyl, and a Boc group. The Boc group means a tert-butoxycarbonyl group.

Examples of an aralkyloxycarbonyl group having 8 to 20 carbon atoms include benzyloxycarbonyl and an Fmoc group. The Fmoc group means a 9-fluorenylmethoxycarbonyl group.

Examples of "amino protective group (protective group of the amino group)" include a formyl group, an acyl group having 1 to 6 carbon atoms (for example, an acetyl group and a propionyl group), an alkoxycarbonyl group having 2 to 6 carbon atoms, a benzoyl group, an arylalkyloxycarbonyl group having 8 to 20 carbon atoms, a trityl group, a monomethoxytrityl group, a 1-(4,4-dimethyl-2,6-dioxocyclohex-1-ylidene)-3-methylbutyl group, a phtaloyl group, an N,N-dimethylaminomethylene group, a silyl group, and an alkenyl group having 2 to 6 carbon atoms. These groups may be substituted with one to three substituents selected from the group consisting of a halogen atom, an alkoxy group having 1 to 6 carbon atoms, and a nitro group.

As the amino protective group, an alkoxycarbonyl group having 2 to 6 carbon atoms or an arylalkyloxycarbonyl group having 8 to 20 carbon atoms is preferable.

Examples of "protective group of the hydroxy group" include an alkyl group having 1 to 6 carbon atoms, a phenyl group, a trityl group, a formyl group, an acyl group having 1 to 6 carbon atoms, a benzoyl group, an arylalkylcarbonyl group having 7 to 10 carbon atoms, a 2-tetrahydropyranyl group, a 2-tetrahydrofuranyl group, a silyl group, and an alkenyl group having 2 to 6 carbon atoms. These groups may be substituted with one to three substituents selected from the group consisting of a halogen atom, an alkyl group having 1 to 6 carbon atoms, an alkoxy group having 1 to 6 carbon atoms, and a nitro group.

Examples of "protective group of the carboxy group" include an alkyl group having 1 to 6 carbon atoms, an arylalkyl group having 7 to 10 carbon atoms, a phenyl group, a trityl group, a silyl group, and an alkenyl group having 2 to 6 carbon atoms. These groups may be substituted with one to three substituents selected from the group consisting of a halogen atom, an alkoxy group having 1 to 6 carbon atoms, and a nitro group.

Examples of "protective group of the carbonyl group" include cyclic acetals (for example, 1,3-dioxane) and acyclic acetals (for example, di(alkyl having 1 to 6 carbon atoms) acetal).

Examples of "protective group of the guanidyl group" include a 2,2,4,6,7-pentamethyldihydrobenzofuran-5-sulfonyl group, a 2,3,4,5,6-pentamethylbenzenesulfonyl group, a tosyl group, and a nitro group.

Examples of "protective group of the mercapto group (sulfhydryl group)" include a trityl group, a 4-methylbenzyl group, an acetylaminomethyl group, a t-butyl group, and a t-butylthio group.

### (Method for producing peptide compound)

The method for producing a peptide compound according to the present disclosure includes a step of using a substituted benzyl compound represented by Formula (1).

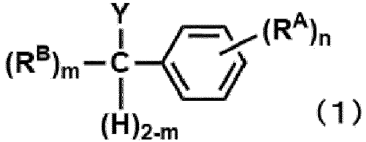

In Formula (1), Y represents -OH, -NHR, -SH, or -X, R represents a hydrogen atom, an alkyl group, an arylalkyl group, a heteroarylalkyl group, or a 9-fluorenylmethoxycarbonyl group, X represents Cl, Br, or I, m represents 1 or 2, n represents an integer of 1 to 5,
R^{B}'s each independently represent an aliphatic hydrocarbon group, and
R^{A}'s each independently represent an aliphatic hydrocarbon group or an organic group having an aliphatic hydrocarbon group, where the number of carbon atoms in at least one aliphatic hydrocarbon group is 12 or more, and a benzene ring in Formula (1) may further have a substituent in addition to R^{A}.

In the substituted benzyl compound represented by Formula (1), R^{B}'s may be each independently a compound having an aromatic group (excluding a phenyl group).

The compound represented by Formula (1) according to the present disclosure has an aliphatic hydrocarbon group having 12 or more carbon atoms, and thus has excellent solubility in a hydrophobic solvent. Furthermore, in a hydrophilic solvent, since the aliphatic hydrocarbon groups in R^{A}'s aggregate with each other, and benzylbenzene rings cause π-π interaction (π-π stacking) between the benzylbenzene rings, crystallization property is excellent, and purification and separation properties are also excellent. In other words, in a case where a compound protected by the substituted benzyl compound represented by Formula (1) is subjected to a reaction, since the compound has excellent solubility in a hydrophobic solvent as a reaction solvent, it is presumed that the reaction proceeds rapidly, and since a target product is efficiently crystallized and purified by adding a polar solvent which is a poor solvent during purification, it is presumed that yield of the obtained compound (peptide compound and the like) is excellent.

The above-described effect is exhibited with more excellent in a case where the number of carbon atoms in the aliphatic hydrocarbon group is 18 or more. The reason is considered that, as the number of carbon atoms increases, the contribution ratio of hydrophobicity in the entire molecule increases, which makes it easier to dissolve in a hydrophobic solvent, and with regard to a hydrophilic solvent, presumed that, as the number of carbon atoms increases, the cohesive force increases, which makes it easier to be crystallized.

In addition, by having R^{B}, excellent side reaction suppressiveness in an amino acid extending step is exhibited. That is, in a case where the compound (the following structural formula) disclosed in WO2007/034812A is used in the sequential reaction of peptide synthesis, there is a sequence in which diketopiperazine is by-produced during a protection of the N-terminal end protective group of the second residue, and the yield and the quality of the peptide are significantly reduced, which is a problem in terms of general-purpose properties. In particular, in a case of a sequence including proline or N-methylated amino acid in the first residue, the tendency is remarkable. In the compound represented by Formula (1) of the present disclosure, by having a substituent having a larger steric hindrance than a hydrogen atom in R^{B}, the effect of suppressing the by-product of the diketopiperazine is excellent.

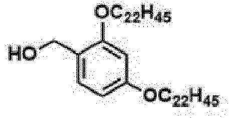

Hereinafter, the method for producing a peptide compound according to the present disclosure will be described in detail.

In the method for producing a peptide compound according to the present disclosure, the substituted benzyl compound represented by Formula (1) can be used not only for formation of a protective group, but also for denaturation of a peptide compound, adjustment of solubility in water or an organic solvent, improvement of crystallinity, multimerization, and the like.

Among these, the substituted benzyl compound represented by Formula (1) is preferably used for formation of a protective group, and more preferably used for forming a C-terminal protective group in an amino acid compound or a peptide compound.

### <Substituted benzyl compound represented by Formula (1)>

The substituted benzyl compound represented by Formula (1) according to the present disclosure (hereinafter, also simply referred to as "compound represented by Formula (1)") is shown below.

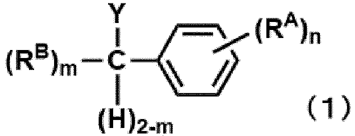

In Formula (1), Y represents -OH, -NHR, -SH, or -X, R represents a hydrogen atom, an alkyl group, an arylalkyl group, a heteroarylalkyl group, or a 9-fluorenylmethoxycarbonyl group, X represents Cl, Br, or I, m represents 1 or 2, n represents an integer of 1 to 5,
R^{B}'s each independently represent an aliphatic hydrocarbon group, and
R^{A}'s each independently represent an aliphatic hydrocarbon group or an organic group having an aliphatic hydrocarbon group, where the number of carbon atoms in at least one aliphatic hydrocarbon group is 12 or more, and a benzene ring in Formula (1) may further have a substituent in addition to R^{A}.

In the substituted benzyl compound represented by Formula (1), R^{B}'s may be each independently a compound having an aromatic group (excluding a phenyl group). The groups other than R^{B} are the same as described above, and the aromatic group may have a substituent.

The aromatic group R^{B} is preferably an aryl group or a heteroaryl group, more preferably an aryl group having 10 or more carbon atoms, and particularly preferably a naphthalene group. The heteroaryl group is preferably monocyclic or bicyclic. The heteroaryl group is more preferably a heteroaryl group having a pyridine ring, a pyrazine ring, a triazine ring, a benzothiophene ring, a furan ring, a benzofuran ring, a pyrrole ring, an indole ring, a carbazole ring, a pyrazole ring, an indazole ring, or a thiophene ring.

From the viewpoint of deprotection rate, solubility in a solvent, and yield, Y in Formula (1) is preferably -OH, -NHR, or -SH, and more preferably -OH or -NHR. In addition, from the viewpoint of mild reaction conditions, Y is preferably -OH or -SH, and more preferably -OH.

R is preferably a hydrogen atom, an amino protective group, an alkyl group having 1 to 6 carbon atoms, or an arylalkyl group having 7 to 16 carbon atoms, more preferably a hydrogen atom, a methyl group, an ethyl group, or a benzyl group, and still more preferably a hydrogen atom.

From the viewpoint of deprotection rate, solubility in a solvent, and yield, m, which is the number of substitutions of R^{B} in Formula (1), is 1 or 2, preferably 1.

R^{B} is preferably an alkyl group having 1 to 6 carbon atoms or an arylalkyl group having 7 to 16 carbon atoms, more preferably a methyl group, an ethyl group, or a benzyl group, still more preferably a methyl group or an ethyl group, and particularly preferably a methyl group.

From the viewpoint of deprotection rate, solubility in a solvent, and yield, n, which is the number of substitutions of R^{A} on the benzene ring in Formula (1), is an integer of 1 to 5, preferably an integer of 1 to 4, more preferably 2 or 3, and particularly preferably 3.

R^{A}'s each independently represent an aliphatic hydrocarbon group or an organic group having an aliphatic hydrocarbon group. In the present specification, the "organic group having an aliphatic hydrocarbon group" in R^{A} is a monovalent (one bonding site bonded to the benzene ring) organic group having an aliphatic hydrocarbon group in its molecular structure.

The "aliphatic hydrocarbon group" or the "aliphatic hydrocarbon group" in the "organic group having an aliphatic hydrocarbon group" is a linear, branched, or cyclic saturated or unsaturated aliphatic hydrocarbon group, and an aliphatic hydrocarbon group having 5 or more carbon atoms is preferable, an aliphatic hydrocarbon group having 5 to 60 carbon atoms is more preferable, an aliphatic hydrocarbon group having 5 to 30 carbon atoms is still more preferable, and an aliphatic hydrocarbon group having 10 to 30 carbon atoms is particularly preferable. The lower limit value of the number of carbon atoms in the aliphatic hydrocarbon group is preferably 12, more preferably 15, and still more preferably 18. The upper limit value thereof is preferably 30, more preferably 26, and still more preferably 24.

The moiety of the "aliphatic hydrocarbon group" in the "organic group having an aliphatic hydrocarbon group" is not particularly limited, and may be present at the terminal (a monovalent group) or may be present at any other site (for example, a divalent group).

Examples of the "aliphatic hydrocarbon group" include an alkyl group, a cycloalkyl group, an alkenyl group, and an alkynyl group.

The "alkyl group" preferably has 5 to 30 carbon atoms, more preferably has 12 to 30 carbon atoms, still more preferably has 16 to 26 carbon atoms, and even more preferably has 18 to 24 carbon atoms. Specific examples thereof include a pentyl group, a hexyl group, an octyl group, a 2-ethylhexyl group, a decyl group, a hexadecyl group, an octadecyl group, an icosyl group, a docosyl group, a tetracosyl group, a lauryl group, a tridecyl group, a myristyl group, and an isostearyl group. Among these, an octadecyl group, an icosyl group, a docosyl group, or a tetracosyl group is preferable, and an icosyl group, a docosyl group, or a tetracosyl group is more preferable.

As the above-described "cycloalkyl group", for example, a cycloalkyl group having 5 to 30 carbon atoms, or the like is preferable, and examples thereof include a cyclopentyl group, a cyclohexyl group, an isobornyl group, and a tricyclodecanyl group. In addition, these may be linked repeatedly, or may be a fused-ring structure of two or more rings.

The "alkenyl group" preferably has 5 to 30 carbon atoms, more preferably has 12 to 30 carbon atoms, still more preferably has 16 to 26 carbon atoms, and even more preferably has 18 to 24 carbon atoms. Specific examples thereof include a pentenyl group, a hexenyl group, and an oleyl group.

The "alkynyl group" preferably has 5 to 30 carbon atoms, more preferably has 12 to 30 carbon atoms, still more preferably has 16 to 26 carbon atoms, and even more preferably has 18 to 24 carbon atoms. Specific examples thereof include a 4-pentenyl group and a 5-hexenyl group.

As the "steroid group", for example, a group having a cholesterol structure, a group having an estradiol structure, or the like is preferable.

The above-described organic group may be further substituted with one or more substituents selected from a silyl group, a hydrocarbon group having a silyloxy structure, and an organic group having a perfluoroalkyl structure.

As the above-described silyl group, a trialkylsilyl group is preferable, and a silyl group having three alkyl groups having 1 to 3 carbon atoms is more preferable.

As the silyloxy structure in the above-described hydrocarbon group having a silyloxy structure, a trialkylsilyloxy structure is preferable, and a silyloxy structure having three alkyl groups having 1 to 3 carbon atoms is more preferable.

The above-described hydrocarbon group having a silyloxy structure preferably has 1 to 3 silyloxy structures.

The number of carbon atoms in the above-described hydrocarbon group having a silyloxy structure is preferably 10 or more, more preferably 10 to 100, and particularly preferably 16 to 50.

Preferred examples of the above-described hydrocarbon group having a silyloxy structure include a group represented by Formula (Si).

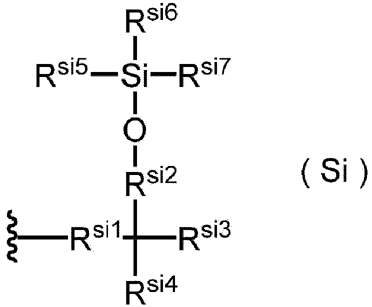

In Formula (Si), R^{sil} represents a single bond or an alkylene group having 1 to 3 carbon atoms, R^{si2} represents an alkylene group having 1 to 3 carbon atoms, R^{si3} and R^{si4} each independently represent a hydrogen atom, an alkyl group having 1 to 6 carbon atoms, or -OSiR^{si5}R^{si6}R^{si7}, and R^{si5} to R^{si7} each independently represent an alkyl group having 1 to 6 carbon atoms or an aryl group.

R^{si5} to R^{si7} in Formula (Si) are each independently preferably an alkyl group having 1 to 6 carbon atoms or a phenyl group, more preferably an alkyl group having 1 to 6 carbon atoms, and particularly preferably a linear or branched alkyl group having 1 to 4 carbon atoms.

As the perfluoroalkyl structure in the above-described organic group having a perfluoroalkyl structure, a perfluoroalkyl structure having 1 to 20 carbon atoms is preferable, a perfluoroalkyl structure having 5 to 20 carbon atoms is more preferable, and a perfluoroalkyl structure having 7 to 16 carbon atoms is particularly preferable. In addition, the above-described perfluoroalkyl structure may be linear, may have a branch, or may have a ring structure.

The above-described organic group having a perfluoroalkyl structure is preferably a perfluoroalkyl group, an alkyl group having a perfluoroalkyl structure, or an alkyl group having a perfluoroalkyl structure and an amide bond in the alkyl chain.

The number of carbon atoms in the above-described organic group having a perfluoroalkyl structure is preferably 5 or more, more preferably 10 or more, still more preferably 10 to 100, and particularly preferably 16 to 50.

Preferred examples of the above-described organic group having a perfluoroalkyl structure include groups shown below.

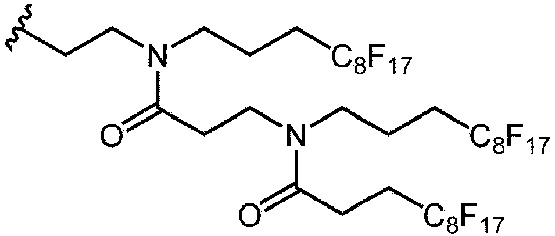

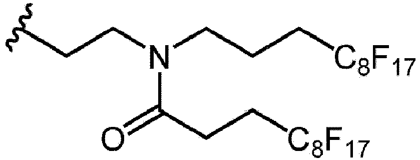

A moiety other than the "aliphatic hydrocarbon group" in the "organic group having an aliphatic hydrocarbon group" can be optionally set. For example, the "organic group having an aliphatic hydrocarbon group" may have a moiety such as -O-, -S-, -COO-, -OCONH-, -CONH-, and a hydrocarbon group (monovalent group or divalent group) other than the "aliphatic hydrocarbon group".

Examples of the "hydrocarbon group" other than the "aliphatic hydrocarbon group" include an aromatic hydrocarbon group, and specifically, for example, a monovalent group such as an aryl group or a divalent group derived from the monovalent group is used.

In addition, the above-described aliphatic hydrocarbon group and the hydrocarbon group other than the above-described aliphatic hydrocarbon group may be substituted with a substituent selected from a halogen atom, an oxo group, and the like.

The bond (substitution) of the "organic group having an aliphatic hydrocarbon group" to the benzene ring may be through the above-described "aliphatic hydrocarbon group" or the above-described "hydrocarbon group" existing in R^{A}, that is, may be directly bonded by a carbon-carbon bond, or may be through a moiety such as -O-, -S-, -COO-, -OCONH-, and -CONH-, which exists in R^{A}. From the viewpoint of ease of synthesizing the compound, it is preferable to be through -O-, -S-, -COO-, or -CONH-, and it is particularly preferable to be through -O-.

In the compound represented by Formula (1) according to the present disclosure, from the viewpoint of solubility in a solvent, crystallization property, and yield, the total number of carbon atoms in all aliphatic hydrocarbon groups included in all R^{A}'s is preferably 24 or more, more preferably 24 to 200, still more preferably 32 to 100, particularly preferably 34 to 80, and most preferably 36 to 80.

In addition, the compound represented by Formula (1) according to the present disclosure is a compound which has at least one aliphatic hydrocarbon group having 12 or more carbon atoms in at least one R^{A}. From the viewpoint of solubility in a solvent, crystallization property, and yield, a compound which has at least one aliphatic hydrocarbon group having 12 to 100 carbon atoms in at least one R^{A} is preferable, a compound which has at least one aliphatic hydrocarbon group having 18 to 40 carbon atoms in at least one R^{A} is more preferable, and a compound which has at least one aliphatic hydrocarbon group having 20 to 36 carbon atoms in at least one R^{A} is still more preferable.

From the viewpoint of crystallization property and yield, the above-described aliphatic hydrocarbon group is preferably an alkyl group and more preferably a linear alkyl group.

In addition, from the viewpoint of solubility in a solvent, crystallization property, and yield, the number of carbon atoms in one R^{A} is preferably 12 to 200, more preferably 18 to 150, still more preferably 18 to 100, and particularly preferably 20 to 80, respectively.

In Formula (1), from the viewpoint of solubility in a solvent, crystallization property, and yield, it is preferable that at least one R^{A} is a group represented by any of Formula (f1), Formula (a1), Formula (b1), or Formula (e1), it is more preferable to be a group represented by Formula (f1) or Formula (a1), and it is particularly preferable to be a group represented by Formula (f1).

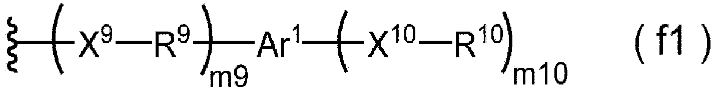

In Formula (f1), a wavy line portion represents a bonding position to a benzene ring, m9 represents an integer of 1 to 3, X⁹'s each independently represent a single bond, -O-, -S-, -COO-, -OCO-, -OCONH-, -NHCONH-, -NHCO-, or -CONH-, R⁹'s each independently represent a divalent aliphatic hydrocarbon group, Ar¹ represents an (m10+1)-valent aromatic group or an (m10+1)-valent heteroaromatic group, m10 represents an integer of 1 to 3, X¹⁰'s each independently represent a single bond, -O-, -S-, -COO-, -OCO-, -OCONH-, -NHCONH-, -NHCO-, or -CONH-, and R¹⁰'s each independently represent a monovalent aliphatic hydrocarbon group having 5 or more carbon atoms.

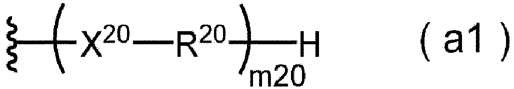

In Formula (a1), a wavy line portion represents a bonding position to a benzene ring, m20 represents an integer of 1 to 10, X²⁰'s each independently represent a single bond, -O-, -S-, -COO-, -OCO-, -OCONH-, -NHCONH-, -NHCO-, or -CONH-, and R²⁰'s each independently represent a divalent aliphatic hydrocarbon group.

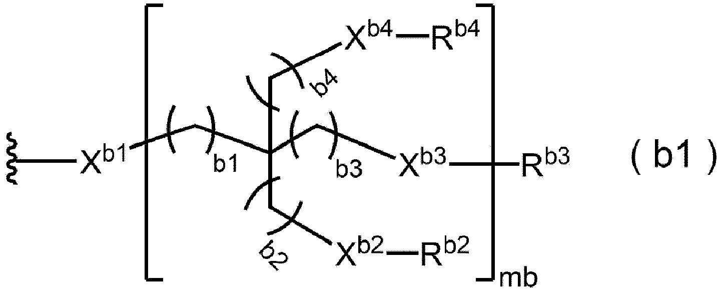

In Formula (b1), a wavy line portion represents a bonding position to the benzene ring, mb represents 1 or 2, b1, b2, b3, and b4 each independently represent an integer of 0 to 2, X^{b1}, X^{b2}, X^{b3}, and X^{b4} each independently represent a single bond, -O-, -S-, -COO-, -OCONH-, or -CONH-, R^{b2} and R^{b4} each independently represent a hydrogen atom, a methyl group, or an aliphatic hydrocarbon group having 5 or more carbon atoms, and R^{b3} represents an aliphatic hydrocarbon group having 5 or more carbon atoms.

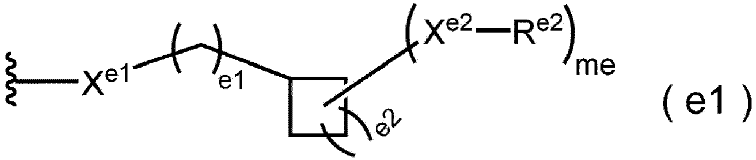

In Formula (e1), a wavy line portion represents a bonding position to a benzene ring, X^{e1} represents a single bond, -O-, -S-, -NHCO-, or -CONH-, me represents an integer of 0 to 15, e1 represents an integer of 0 to 11, e2 represents an integer of 0 to 5, X^{e2}'s each independently represent a single bond, -O-, -S-, -COO-, -OCONH-, -NHCO-, or -CONH-, and R^{e2}'s each independently represent a hydrogen atom, a methyl group, or an organic group having an aliphatic hydrocarbon group having 5 or more carbon atoms.

m9 in Formula (f1) is preferably 1 or 2 and more preferably 1.

X⁹ and X¹⁰ in Formula (f1) are each independently preferably -O-, -S-, -COO-, -OCONH-, or -CONH-, and more preferably -O-.

R⁹'s in Formula (f1) are each independently preferably an alkylene group having 1 to 10 carbon atoms, more preferably an alkylene group having 1 to 4 carbon atoms, and particularly preferably a methylene group.

R¹⁰'s in Formula (f1) are each independently preferably a monovalent aliphatic hydrocarbon group having 5 to 60 carbon atoms, more preferably a monovalent aliphatic hydrocarbon group having 12 to 50 carbon atoms, still more preferably a monovalent aliphatic hydrocarbon group having 18 to 40 carbon atoms, and particularly preferably a monovalent aliphatic hydrocarbon group having 20 to 32 carbon atoms. In addition, R¹⁰'s are each independently preferably a linear alkyl group or a branched alkyl group and more preferably a linear alkyl group.

m10 in Formula (f1) is preferably 2 or 3 and more preferably 2.

Ar¹ in Formula (f1) is preferably an (m10+1)-valent aromatic group, more preferably a group obtained by removing (m10+1) pieces of hydrogen atoms from benzene or a group obtained by removing (m10+1) pieces of hydrogen atoms from benzene, and particularly preferably a group obtained by removing (m10+1) pieces of hydrogen atoms from benzene.

In addition, from the viewpoint of solubility in a solvent, crystallization property, and yield, the group represented by Formula (f1) is preferably a group represented by Formula (f2).

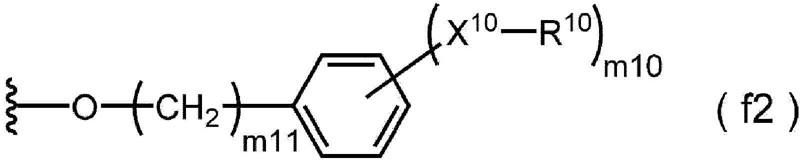

In Formula (f2), a wavy line portion represents a bonding position to a benzene ring, m10 represents an integer of 1 to 3, m11 represents an integer of 1 to 3, X¹⁰'s each independently represent a single bond, -O-, -S-, -COO-, -OCO-, -OCONH-, -NHCONH-, -NHCO-, or -CONH-, and R¹⁰'s each independently represent a monovalent aliphatic hydrocarbon group having 5 or more carbon atoms.

m10, X¹⁰, and R¹⁰ in Formula (f2) have the same meanings as m10, X¹⁰, and R¹⁰ in Formula (f1), respectively, and the preferred aspects thereof are also the same.

m11 in Formula (f2) is preferably 1 or 2 and more preferably 1.

m20 in Formula (a1) is preferably 1 or 2 and more preferably 1.

X²⁰'s in Formula (a1) are each independently preferably -O-, -S-, -COO-, -OCONH-, or -CONH-, and more preferably -O-.

R²⁰ in Formula (a1) is preferably a divalent aliphatic hydrocarbon group having 5 or more carbon atoms, more preferably a divalent aliphatic hydrocarbon group having 5 to 60 carbon atoms, still more preferably a divalent aliphatic hydrocarbon group having 8 to 40 carbon atoms, and particularly preferably a divalent aliphatic hydrocarbon group having 12 to 32 carbon atoms. In addition, R²⁰ is preferably a linear alkylene group.

mb in Formula (b1) is preferably 1.

b1 to b4 in Formula (b1) are each independently preferably 1 or 2 and more preferably 1.

X^{b1} to X^{b4} in Formula (b1) are each independently preferably -O-, -S-, -COO-, -OCONH-, or -CONH-, and more preferably -O-.

R^{b2} and R^{b4} in Formula (b1) are each independently preferably a hydrogen atom, a methyl group, or an aliphatic hydrocarbon group having 5 to 60 carbon atoms, more preferably a hydrogen atom, a methyl group, or an alkyl group having 8 to 40 carbon atoms, and particularly preferably a hydrogen atom, a methyl group, or an alkyl group having 12 to 32 carbon atoms.

R^{b3} in Formula (b1) is preferably a monovalent aliphatic hydrocarbon group having 5 to 60 carbon atoms, more preferably a monovalent aliphatic hydrocarbon group having 5 to 60 carbon atoms, still more preferably a monovalent aliphatic hydrocarbon group having 8 to 40 carbon atoms, and particularly preferably a monovalent aliphatic hydrocarbon group having 12 to 32 carbon atoms. In addition, R^{b3} is preferably a linear alkyl group.

In the compound represented by Formula (1) according to the present disclosure, from the viewpoint of solubility in a solvent and yield, preferred examples of the aliphatic hydrocarbon group in R^{A} include an aliphatic hydrocarbon group having a branch, and more preferred examples thereof include groups shown below. A wavy line portion represents a bonding position to another structure, nt2 represents an integer of 3 to 74, and nt3 represents an integer set such that the total number of carbon atoms in the following group is 14 to 300.

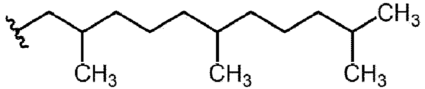

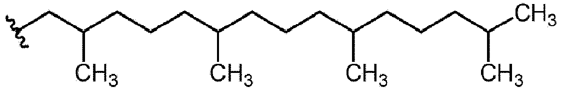

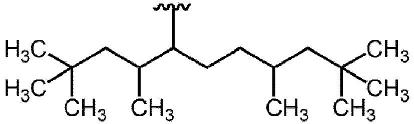

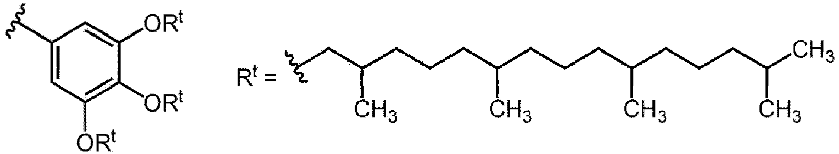

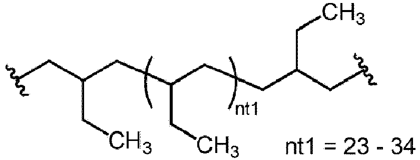

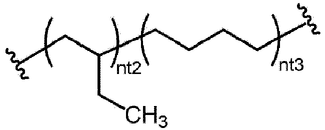

The substituent which may be included in the compound represented by Formula (1) on the benzene ring is not particularly limited, and examples thereof include an alkoxy group, an aryloxy group, a halogen atom, an alkyl group, a halogenated alkyl group, an aryl group, an acyl group, an acyloxy group, an alkoxycarbonyl group, an aryloxycarbonyl group, an alkylthio group, an arylthio group, R^{st}-CO-NR^{st}-, -CON(R^{st})₂, a dialkylamino group, an alkylarylamino group, a diarylamino group, and a group obtained by combining two or more of these groups. R^{st} represents a hydrogen atom, an alkyl group, or an aryl group.

The molecular weight of the compound represented by Formula (1) is not particularly limited, but from the viewpoint of deprotection rate, crystallization property, solubility in a solvent, and yield, it is preferably 340 to 3,000, more preferably 400 to 2,000, still more preferably 500 to 1,500, and particularly preferably 800 to 1,300. In addition, in a case where the molecular weight is 3,000 or less, the proportion of Formula (1) in the target product is appropriate and the proportion of a compound obtained by deprotecting Formula (1) is not reduced, so that productivity is excellent.

Preferred specific examples of the compound represented by Formula (1) include compounds shown below. In the following compounds, R^{B} is as defined above in the present specification. R^{g} represents an aliphatic hydrocarbon group having 12 or more carbon atoms, and an aliphatic hydrocarbon group having 12 to 100 carbon atoms is preferable, an aliphatic hydrocarbon group having 18 to 40 carbon atoms is more preferable, and an aliphatic hydrocarbon group having 20 to 32 carbon atoms is particularly preferable. In addition, the above-described aliphatic hydrocarbon group is preferably a linear alkyl group, a branched alkyl group, or a cyclic alkyl group, and more preferably a linear alkyl group.

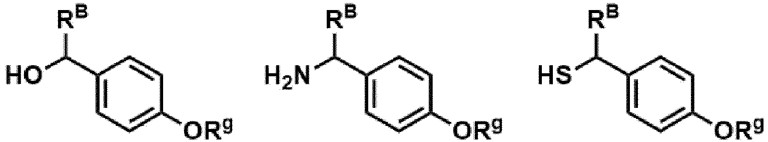

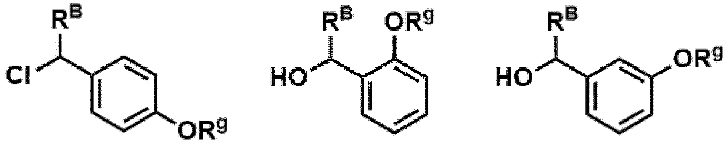

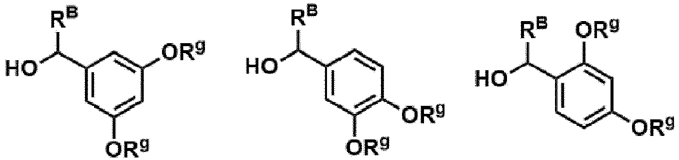

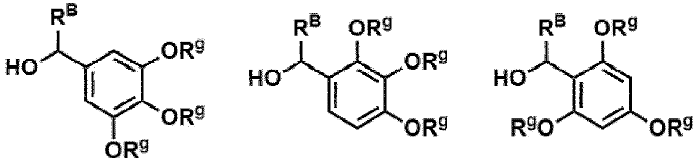

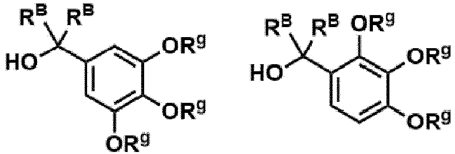

### <Method for producing substituted benzyl compound represented by Formula (1)>

A method for producing the substituted benzyl compound represented by Formula (1) according to the present disclosure is not particularly limited, and can be produced by referring to a known method.

Unless otherwise specified, a raw material compound used for producing the substituted benzyl compound represented by Formula (1) may be a commercially available compound, or may be produced by a known method or a method according to the known method.

In addition, the produced substituted benzyl compound represented by Formula (1) may be purified by a known purification method as necessary. For example, a method of isolating and purifying by recrystallization, column chromatography, or the like, a method of purifying by reprecipitation with a unit for changing the solution temperature, a unit for changing the solution composition, or the like, and the like can be performed.

The method for synthesizing the substituted benzyl compound represented by Formula (1) according to the present disclosure is not particularly limited, but the substituted benzyl compound represented by Formula (1) can be synthesized according to, for example, the following schemes. In addition, it is also possible to synthesize by referring to the synthesis method described in WO2010/113939A.

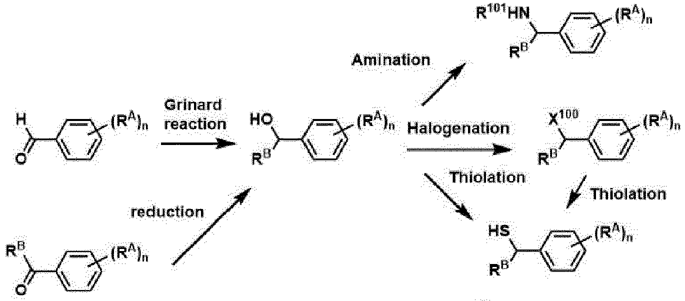

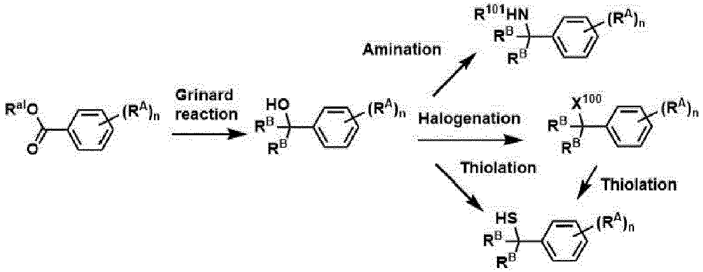

R^{A} and R^{B} are as defined in the present specification.

R^{a1} represents an alkyl group, X¹⁰⁰ represents Cl, Br, or I, and R¹⁰¹ represents a hydrogen atom, an alkyl group, or an Fmoc group.

In the method for producing a peptide compound according to the present disclosure, it is preferable that the step of using the substituted benzyl compound represented by Formula (1) is a C-terminal protecting step of protecting a carboxy group or an amide group of an amino acid compound or a peptide compound with the substituted benzyl compound represented by Formula (1).

The peptide compound is a generic name of a compound formed by an amide bond or an ester bond of an amino acid or an amino acid analogue. A compound obtained by further chemically modifying the above-described compound is also included in the peptide compound according to the embodiment of the present invention.

An amino acid compound constituting the peptide compound, for example, an amino acid and an amino acid analogue may be referred to as an amino acid residue and an amino acid analogue residue, respectively. The amino acid residue and the amino acid analog residue mean a monovalent or divalent group derived from the amino acid or the amino acid analog.

The amino acid is an α, β, or γ-amino acid, and is not limited to a natural amino acid (specifically, refers to Gly, Ala, Ser, Thr, Val, Leu, Ile, Phe, Tyr, Trp, His, Glu, Asp, Gln, Asn, Cys, Met, Lys, Arg, and Pro), and may be an unnatural amino acid. The unnatural amino acid refers to an amino acid other than the above-described natural amino acid. In a case of the α-amino acid, an L-type amino acid, a D-type amino acid, or an α,α-dialkylamino acid may be used. A side chain of the amino acid may have, for example, a substituent in addition to hydrogen atoms.

In addition, the amino acid may be an amino acid in which a carboxy group is converted into an amide group.

The amino acid analog is preferably α-hydroxycarboxylic acid or α-mercaptocarboxylic acid. Side chains of the α-hydroxycarboxylic acid and the α-mercaptocarboxylic acid may have various substituents in addition to hydrogen atoms, similarly to the amino acid.

From the viewpoint of ease of synthesis of the peptide compound and yield, it is more preferable that the method for producing a peptide compound according to the present disclosure further includes, in addition to the C-terminal protecting step of protecting the carboxy group of the amino acid compound or the peptide compound with the substituted benzyl compound represented by Formula (1), an N-terminal deprotecting step of deprotecting an N-terminal end of an N-terminal and C-terminal protected amino acid compound or an N-terminal and C-terminal protected peptide compound, which is obtained in the C-terminal protecting step; and a peptide chain extending step of condensing the N-terminal end of a C-terminal protected amino acid compound or a C-terminal protected peptide compound, which is obtained in the N-terminal deprotecting step, with an N-terminal protected amino acid compound or an N-terminal protected peptide compound.

It is still more preferable that the method for producing a peptide compound according to the present disclosure further includes a precipitating step of precipitating the N-terminal and C-terminal protected peptide compound obtained in the peptide chain extending step.

It is particularly preferable that the method for producing a peptide compound according to the present disclosure further includes, one or more times in the following order after the precipitating step, a step of deprotecting an N-terminal end of the obtained N-terminal and C-terminal protected peptide compound, a step of condensing the N-terminal end of the obtained C-terminal protected peptide compound with an N-terminal protected amino acid compound or an N-terminal protected peptide compound, and a step of precipitating the obtained N-terminal and C-terminal protected peptide compound.

It is preferable that the method for producing a peptide compound according to the present disclosure further includes a C-terminal deprotecting step of deprotecting a C-terminal protective group.

It is preferable that the method for producing a peptide compound according to the present disclosure further includes, before the above-described C-terminal protecting step, a dissolving step of dissolving the substituted benzyl compound represented by Formula (1) in a solvent.

Hereinafter, each step and the like described above will be described in detail.

### <Dissolving step>

It is preferable that the method for producing a peptide compound according to the present disclosure includes, before the above-described C-terminal protecting step, a dissolving step of dissolving the substituted benzyl compound represented by Formula (1) in a solvent.

As the solvent, a general organic solvent can be used for the reaction, but since excellent reactivity can be expected as solubility of the substituted benzyl compound represented by Formula (1) in the above-described solvent is higher, it is preferable to select a solvent having a high solubility with the substituted benzyl compound represented by Formula (1). Specific examples thereof include halogenated hydrocarbons such as chloroform and dichloromethane; and nonpolar organic solvents such as 1,4-dioxane, tetrahydrofuran, and cyclopentyl methyl ether. Two or more of these solvents may be mixed and used in an appropriate ratio. In addition, as long as the substituted benzyl compound represented by Formula (1) can be dissolved, in the above-described halogenated hydrocarbons or nonpolar organic solvents, aromatic hydrocarbons such as benzene, toluene, and xylene; nitriles such as acetonitrile and propionitrile; ketones such as acetone and 2-butanone; amides such as N,N-dimethylformamide and N-methylpyrrolidone; and sulfoxides such as dimethyl sulfoxide may be mixed and used in an appropriate ratio.

In addition, a solvent described in Organic Process Research & Development, 2017, 21, 3, 365 to 369 may be used.

### <C-terminal protecting step>

It is preferable that the method for producing a peptide compound according to the present disclosure includes a C-terminal protecting step of protecting a carboxy group of an amino acid compound or a peptide compound with the substituted benzyl compound represented by Formula (1).

The amino acid compound or peptide compound used in the above-described C-terminal protecting step is not particularly limited, and a known compound can be used. However, an N-terminal protected amino acid compound or an N-terminal protected peptide compound is preferable, and an Fmoc-protected amino acid compound or an Fmoc-protected peptide compound is more preferable.

In addition, it is preferable that a hydroxy group, an amino group, a carbonyl group, an amide group, an imidazole group, an indole group, a guanidyl group, a mercapto group, or the like, which is a moiety other than the C-terminal end in the amino acid compound or peptide compound used in the above-described C-terminal protecting step, is protected by a known protective group such as a protective group described later.

The amount of the amino acid compound or peptide compound, which is used as a reaction substrate, to be used is preferably 1 molar equivalent to 10 molar equivalent, more preferably 1 molar equivalent to 5 molar equivalent, still more preferably 1 molar equivalent to 2 molar equivalent, and particularly preferably 1 molar equivalent to 1.5 molar equivalent with respect to 1 molar equivalent of the substituted benzyl compound represented by Formula (1).

In a case where a substituted benzyl compound represented by Formula (1), in which Y in Formula (1) is -OH or -SH, is used, it is preferable to add a condensing agent in the presence of a condensation additive (condensation accelerator) in a solvent which does not affect the reaction, or to react in an acid catalyst.

In a case where a substituted benzyl compound represented by Formula (1), in which Y in Formula (1) is -NHR, is used, it is preferable to add a condensing agent in the presence of a condensation additive (condensation accelerator).

The amount of the condensation additive to be used is preferably 0.05 molar equivalent to 1.5 molar equivalent with respect to 1 molar equivalent of the substituted benzyl compound represented by Formula (1).

As the condensing agent, a condensing agent generally used in peptide synthesis can be used without limitation in the present disclosure. Examples thereof include 4-(4,6-dimethoxy-1,3,5-triazin-2-yl)-4-methylmorpholinium chloride (DMTMM), O-(benzotriazol-1-yl)-1,1,3,3-tetramethyluronium hexafluorophosphate (HBTU), O-(7-azabenzotriazol-1-yl)-1,1,3,3-tetramethyluronium hexafluorophosphate (HATU), O-(6-chlorobenzotriazol-1-yl)-1,1,3,3-tetramethyluronium hexafluorophosphate (HBTU(6-Cl)), O-(benzotriazol-1-yl)-1,1,3,3-tetramethyluronium tetrafluoroborate (TBTU), O-(6-chlorobenzotriazol-1-yl)-1,1,3,3-tetramethyluronium tetrafluoroborate (TCTU), (1-cyano-2-ethoxy-2-oxoethylidenaminooxy)dimethylamino-morpholino-carbenium hexafluorophosphate (COMU), dicyclohexylcarbodiimide (DCC), diisopropylcarbodiimide (DIC), 1-ethyl-3-(3-dimethylaminopropyl) carbodiimide (EDC), a hydrochloride salt (EDC/HCl) of 1-ethyl-3-(3-dimethylaminopropyl) carbodiimide, and (benzotriazol-1-yloxy)tripyrrolidinophosphonium hexafluorophosphate (PyBoP), but the condensing agent is not limited thereto.

Among these, DIC, EDC, EDC/HCl, DMTMM, HBTU, HATU, or COMU is preferable.

The amount of the condensing agent to be used is preferably 1 molar equivalent to 10 molar equivalent and more preferably 1 molar equivalent to 5 molar equivalent with respect to 1 molar equivalent of the substituted benzyl compound represented by Formula (1).

As the acid catalyst used in the condensation reaction, an acid catalyst generally used in peptide synthesis can be used without limitation.

The amount of the acid catalyst to be used is preferably more than 0 molar equivalent and 4.0 molar equivalent or less, more preferably 0.05 molar equivalent to 1.5 molar equivalent, and still more preferably 0.1 molar equivalent to 0.3 molar equivalent with respect to 1 molar equivalent of the substituted benzyl compound represented by Formula (1).

As the acid catalyst used in the condensation reaction, an acid catalyst generally used in peptide synthesis can be used without limitation, and examples thereof include methanesulfonic acid, trifluoromethanesulfonic acid, and p-toluenesulfonic acid.

Among these, methanesulfonic acid or p-toluenesulfonic acid is preferable.

The amount of the acid catalyst to be used is preferably more than 0 molar equivalent and 4.0 molar equivalent or less, more preferably 0.05 molar equivalent to 1.5 molar equivalent, and still more preferably 0.1 molar equivalent to 0.3 molar equivalent with respect to 1 molar equivalent of the substituted benzyl compound represented by Formula (1).

In the above-described C-terminal protecting step, it is preferable to add the condensation accelerator in order to promote the reaction and suppress side reactions such as racemization.

The condensation accelerator in the present disclosure is a reagent which, in a case of coexisting with the condensing agent, leads an amino acid to a corresponding active ester, symmetric acid anhydride, or the like to facilitate the formation of a peptide bond (amide bond).

As the condensation accelerator, a condensation accelerator generally used in peptide synthesis can be used without limitation, and examples thereof include 4-dimethylaminopyridine, N-methylimidazole, boronic acid derivative, 1-hydroxybenzotriazole (HOBt), ethyl 1-hydroxytriazole-4-carboxylate (HOCt), 1-hydroxy-7-azabenzotriazole (HOAt), 3-hydroxy-1,2,3-benzotriazin-4(3H)-one (HOOBt), N-hydroxysuccinimide (HOSu), N-hydroxyphthalimide (HOPht), N-hydroxy-5-norbornene-2,3-dicarboxyimide (HONb), pentafluorophenol, and ethyl(hydroxyimino)cyanoacetylate (Oxyma). Among these, 4-dimethylaminopyridine, HOBt, HOCt, HOAt, HOOBt, HOSu, HONb, or Oxyma is preferable.

The amount of the condensation accelerator to be used is preferably more than 0 molar equivalent and 4.0 molar equivalent or less and more preferably 0.1 molar equivalent to 1.5 molar equivalent with respect to the amino acid compound or peptide compound.

As the solvent, the above-described solvent used in the above-described dissolving step can be suitably used.

The reaction temperature is not particularly limited, but is preferably -10°C to 50°C and more preferably 0°C to 40°C. The reaction time is not particularly limited, but is preferably 1 hour to 30 hours.

To confirm the progress of the reaction, a method same as that of a general liquid phase organic synthesis reaction can be applied. That is, the reaction can be traced using thin-layer silica gel chromatography, high performance liquid chromatography, nuclear magnetic resonance (NMR), or the like.

The N-terminal and C-terminal protected amino acid compound or N-terminal and C-terminal protected peptide compound obtained by the above-described C-terminal protecting step may be purified.

For example, in order to isolate the product obtained after dissolving the obtained N-terminal and C-terminal protected amino acid compound or N-terminal and C-terminal protected peptide compound in a solvent and performing a desired organic synthesis reaction, it is preferable to perform a method of changing the solvent to a solvent in which the N-terminal and C-terminal protected amino acid compound or N-terminal and C-terminal protected peptide compound is dissolved (for example, change of solvent composition or change of solvent type) and reprecipitating the resultant.

Specifically, for example, the reaction is performed under conditions such that the N-terminal and C-terminal protected amino acid compound or N-terminal and C-terminal protected peptide compound is dissolved, and after the reaction, the solvent is distilled off and then replaced, or after the reaction, by adding a polar solvent to the reaction system without distilling off the solvent, aggregates are precipitated and impurities are eliminated. As the solvent for replacement, polar organic solvents such as methanol, acetonitrile, and water are used alone or in combination. That is, the reaction is performed under conditions such that the N-terminal and C-terminal protected amino acid compound or N-terminal and C-terminal protected peptide compound is dissolved, and in the solvent replacement after the reaction, for example, a halogenated solvent, tetrahydrofuran (THF), or the like is used for dissolution, and a polar organic solvent such as methanol, acetonitrile, and water is used for precipitation.

### <N-terminal deprotecting step>

It is preferable that the method for producing a peptide compound according to the present disclosure includes an N-terminal deprotecting step of deprotecting an N-terminal end of the N-terminal and C-terminal protected amino acid compound or N-terminal and C-terminal protected peptide compound, which is obtained in the C-terminal protecting step.

As the N-terminal protective group, a protective group for an amino group, which is generally used in technical fields such as peptide chemistry, can be used, but in the present disclosure, a tert-butoxycarbonyl group (hereinafter, also referred to as a Boc group), a benzyloxycarbonyl group (hereinafter, also referred to as a Cbz group or a Z group), or a 9-fluorenylmethoxycarbonyl group (hereinafter, also referred to as an Fmoc group) is suitably used.

The deprotection condition is appropriately selected depending on the type of the temporary protective group, but a group which can be deprotected under conditions different from the removal of the protective group derived from the substituted benzyl compound represented by Formula (1) is preferable. For example, in a case of the Fmoc group, the deprotection is performed by treating with a base, and in a case of the Boc group, the deprotection is performed by treating with an acid. The above-described reaction is performed in a solvent which does not affect the reaction.

Examples of the base include secondary amines such as dimethylamine and diethylamine, and non-nucleophilic organic bases such as 1,8-diazabicyclo[5.4.0]-7-undecene (DBU), 1,4-diazabicyclo[2.2.2]octane (DABCO), and 1,5-diazabicyclo[4.3.0]-5-nonene (DBN).

Examples of the acid include an organic acid which does not affect the condensation reaction, such as trifluoromethanesulfonic acid, methanesulfonic acid, and toluenesulfonic acid.

As the solvent, the above-described solvent used in the above-described dissolving step can be suitably used.

### <Peptide chain extending step>

It is preferable that the method for producing a peptide compound according to the present disclosure includes a peptide chain extending step of condensing the N-terminal end of a C-terminal protected amino acid compound or a C-terminal protected peptide compound, which is obtained in the N-terminal deprotecting step, with an N-terminal protected amino acid compound or an N-terminal protected peptide compound.

The above-described peptide chain extending step is preferably performed under peptide synthesis conditions generally used in the field of peptide chemistry, in which the above-described condensing agent, condensation additive, and the like are used.

The N-terminal protected amino acid compound or N-terminal protected peptide compound is not particularly limited, and a desired compound can be used. However, an Fmoc-protected amino acid compound or an Fmoc-protected peptide compound can be suitably used.

In addition, it is preferable that a hydroxy group, an amino group, a carbonyl group, an amide group, an imidazole group, an indole group, a guanidyl group, a mercapto group, or the like, which is a moiety other than the C-terminal end in the N-terminal protected amino acid compound or N-terminal protected peptide compound, is protected by a known protective group such as a protective group described above.

### <Precipitating step>

It is preferable that the method for producing a peptide compound according to the present disclosure further includes a precipitating step of precipitating the N-terminal and C-terminal protected peptide compound obtained in the peptide chain extending step.

The above-described precipitating step can be performed in the same manner as the precipitation method in the purification which may be performed after the above-described C-terminal protecting step.

### <Chain extension>

It is preferable that the method for producing a peptide compound according to the present disclosure further includes, one or more times in the following order after the precipitating step, a step of deprotecting an N-terminal end of the obtained N-terminal and C-terminal protected peptide compound, a step of condensing the N-terminal end of the obtained C-terminal protected peptide compound with an N-terminal protected amino acid compound or an N-terminal protected peptide compound, and a step of precipitating the obtained N-terminal and C-terminal protected peptide compound.

By repeating the above-described three steps, the chain extension of the obtained peptide compound can be easily performed.

Each step in the above-described three steps can be performed in the same manner as each corresponding step described above.

### <C-terminal deprotecting step>

It is preferable that the method for producing a peptide compound according to the present disclosure further includes a C-terminal deprotecting step of deprotecting a C-terminal protective group.

In the above-described C-terminal deprotecting step, by removing the C-terminal protective group formed by the substituted benzyl compound represented by Formula (1) in the C-terminal protected peptide compound having a desired number of amino acid residues, a peptide compound, which is the final target product, can be obtained.

Preferred examples of a method of removing the C-terminal protective group include a deprotecting method using an acidic compound.

Examples thereof include a method using an acid catalyst and a hydrogenating method using a metal catalyst. Examples of the acid catalyst include trifluoroacetic acid (TFA) and hydrochloric acid, and TFA is preferable. The concentration of TFA can be appropriately selected depending on the protective group and the deprotection condition, and examples thereof include 1% by mass to 100% by mass with respect to the total mass of the solvent used. Examples of the metal catalyst include palladium and platinum oxide.

The peptide compound, which is the final target product obtained by the method for producing a peptide compound according to the present disclosure, can be isolated and purified according to a method commonly used in peptide chemistry. For example, the peptide compound, which is the final target product, can be isolated and purified by extraction and washing the reaction mixture, crystallization, chromatography, and the like.

The type of peptide produced by the method for producing a peptide compound according to the present disclosure is not particularly limited, but the number of amino acid residues of the peptide compound is, for example, preferably 2 to 100, more preferably 2 to 50, and still more preferably 2 to 30. Same as existing or unknown synthetic or native peptides, the peptide obtained by the method for producing a peptide compound according to the present disclosure can be used in various fields such as pharmaceuticals, foods, cosmetics, electronic materials, biosensors, and the like, but the use of the peptide is not limited thereto.

In the method for producing a peptide compound according to the present disclosure, the precipitating step can be appropriately omitted as long as it does not affect the reaction in the next step.

In a case where the amino acid compound or peptide compound used in the method for producing a peptide compound according to the present disclosure has a hydroxy group, an amino group, a carboxy group, a carbonyl group, a guanidyl group, a mercapto group, or the like, a protective group generally used in peptide chemistry or the like may be introduced into these groups, and the target compound can be obtained by removing the protective group as necessary after the reaction.

The method of removing the protective group may be performed according to a known method described in, for example, Protective Groups in Organic Synthesis, John Wiley and Sons (1980). For example, a method using acid, base, ultraviolet light, hydrazine, phenylhydrazine, sodium N-methyldithiocarbamate, tetrabutylammonium fluoride, palladium acetate, trialkylsilyl halide (for example, trimethylsilyl iodide and trimethylsilyl bromide), or the like, a reduction method, and the like are used.

### (Protective group-forming reagent)

The protective group-forming reagent according to the present disclosure includes the above-described substituted benzyl compound represented by Formula (1).

The protective group-forming reagent according to the present disclosure is preferably a protective group-forming reagent of a carboxy group or an amide group, and more preferably a C-terminal protective group-forming reagent of an amino acid compound or a peptide compound.

A preferred aspect of the substituted benzyl compound represented by Formula (1) in the protective group-forming reagent according to the present disclosure is the same as the above-described preferred aspect of the substituted benzyl compound represented by Formula (1) according to the present disclosure.

The protective group-forming reagent according to the present disclosure may be a solid reagent or a liquid reagent.

The content of the substituted benzyl compound represented by Formula (1) in the protective group-forming reagent according to the present disclosure is not particularly limited, but is preferably 0.1% by mass to 100% by mass, more preferably 1% by mass to 100% by mass, and still more preferably 3% by mass to 100% by mass with respect to the total mass of the protective group-forming reagent.

The protective group-forming reagent according to the present disclosure may include a component other than the substituted benzyl compound represented by Formula (1).

As the other components, a known component can be included. Examples thereof include water, an organic solvent, an antioxidant, and a pH adjuster.

### Examples

Hereinafter, the embodiments of the present invention will be more specifically described with reference to Examples. The materials, amounts to be used, proportions, treatment contents, treatment procedures, and the like shown in Examples can be appropriately modified as long as the modifications do not depart from the spirit of the embodiments of the present invention. Therefore, the scope of the embodiments of the present invention is not limited to the following specific examples. In addition, "parts" and "%" are on a mass basis unless otherwise specified.

Unless otherwise specified, purification by column chromatography was performed using an automatic purification device ISOLERA (manufactured by Biotage Ltd.) or a medium pressure liquid chromatograph YFLC-Wprep 2XYN (manufactured by YAMAZEN).

Unless otherwise specified, SNAPKP-SIl Cartridge (manufactured by Biotage Ltd.) or a high flash column WO01, WO02, WO03, WO04, or WO05 (manufactured by YAMAZEN) was used as a carrier in silica gel column chromatography.

The mixing ratio in an eluent used for column chromatography is the volume ratio. For example, "gradient elution of hexane:ethyl acetate = 50:50 to 0:100" means that an eluent of 50% hexane and 50% ethyl acetate is finally changed to an eluent of 0% hexane and 100% ethyl acetate.

In addition, "gradient elution of hexane:ethyl acetate = 50:50 to 0:100 and gradient elution of methanol:ethyl acetate = 0:100 to 20:80" means that an eluent of 50% hexane and 50% ethyl acetate is changed to an eluent of 0% hexane and 100% ethyl acetate, the eluent of 0% hexane and 100% ethyl acetate is switched to an eluent of 0% methanol and 100% ethyl acetate, and then the eluent of 0% methanol and 100% ethyl acetate is finally changed to an eluent of 20% methanol and 80% ethyl acetate.

Mass (MS) spectrum was measured using ACQUITY SQD LC/MS System (manufactured by Waters Corporation; ionization method; electrospray ionization (ESI) method).

NMR spectrum was measured using Bruker AV300 (manufactured by Bruker, 300 MHz) or Bruker AV400 (manufactured by Bruker, 400 MHz), using tetramethylsilane as an internal reference, and all δ values were shown in ppm.

### <Synthesis of protective group-forming reagent (compound (1-1))>

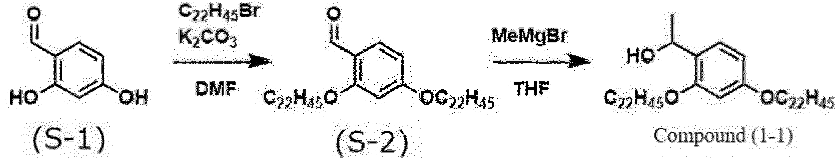

2,4-dihydroxybenzaldehyde (S-1) (1.00 g, 7.24 mmol), 1-bromodocosane (8.46 g, 21.7 mmol), potassium carbonate (6.00 g, 43.4 mmol), and N,N-dimethylformamide (20.0 mL) were mixed and stirred at 80°C for 1.5 hours under a nitrogen atmosphere. After confirming the completion of the reaction, toluene and water were added thereto. The organic layer was separated by an extraction operation, and the obtained organic layer was concentrated under reduced pressure. The methanol was added thereto, the mixture was stirred at 65°C and then allowed to stand to cool to room temperature, and the precipitated solid was collected by filtration. The solid collected by filtration was added to a mixed solution of hexane and methanol= 9:1, stirred at 70°C, and then allowed to cool to room temperature, and the precipitated solid was collected by filtration and dried under reduced pressure to obtain an intermediate (S-2) (4.87 g, yield: 89%).

Under a nitrogen atmosphere, the intermediate (S-2) (1.00 g, 1.32 mmol) and tetrahydrofuran (13.2 mL) were mixed, and a tetrahydrofuran solution of methylmagnesium bromide (1 M (= 1 mol/L)) (1.59 mL, 1.59 mmol) was added dropwise thereto at 35°C. The reaction solution was stirred at 35°C for 1.5 hours, and the completion of the reaction was confirmed. Acetone and a 10% ammonium chloride aqueous solution were slowly added dropwise thereto, and 2-methyltetrahydrofuran was added thereto. The organic layer was separated by an extraction operation, and the obtained organic layer was dried over magnesium sulfate and concentrated under reduced pressure after filtration. After redissolving in tetrahydrofuran (5.00 mL), methanol (50.0 mL) was added thereto, and the precipitated solid was collected by filtration and dried under reduced pressure to obtain a compound (1-1) (0.98 g, yield: 96%).
¹H-NMR (CDCl₃, 300 MHz): δ = 0.88 (6H, t), 1.19 to 1.47 (76H, m), 1.49 (3H, d), 1.73 to 1.84 (4H, m), 2.65 (1H, d), 3.93 (2H, t), 3.97 (2H, t), 4.99 to 5.05 (1H, dq), 6.43 to 6.45 (2H, m), 7.18 (1H, d)

### <Synthesis of protective group-forming reagent (compound (1-2))>

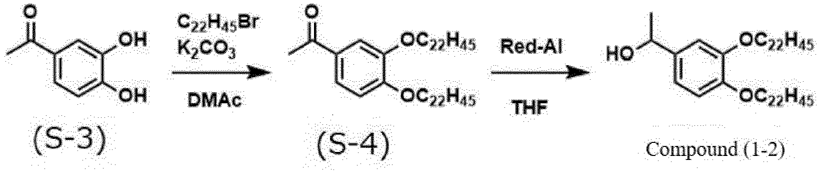

3,4-dihydroxyacetophenone (S-3) (2.00 g, 13.1 mmol), 1-bromodocosane (10.8 g, 27.7 mmol), potassium carbonate (5.5 g, 39.8 mmol), and N,N-dimethylacetamide (50.0 mL) were mixed and stirred at 110°C for 3 hours under a nitrogen atmosphere. After confirming the completion of the reaction, acetic acid (1.1 mL) and water (40.0 mL) were added thereto at 80°C. The mixture was allowed to cool to room temperature, and the precipitated solid was collected by filtration. After washing with water and methanol, an intermediate (S-4) (9.50 g, yield: 94%) was obtained by performing drying under reduced pressure.

The intermediate (S-4) (5.00 g, 6.50 mmol) and tetrahydrofuran (50.0 mL) were mixed under a nitrogen atmosphere, the mixture was stirred at 50°C, and then a 70% toluene solution of sodium bis(2-methoxyethoxy)aluminum hydride (Red-Al) (2.00 mL, 8.13 mmol) was added dropwise thereto. The reaction solution was stirred for 30 minutes, acetone and a 20% aqueous solution of Rochelle salt were added dropwise thereto after the completion of the reaction, and the mixture was allowed to cool to room temperature. The organic layer was separated by an extraction operation, and the obtained organic layer was dried over magnesium sulfate and concentrated under reduced pressure. After redissolving in tetrahydrofuran (5.0 mL), the resultant was added dropwise to methanol (50 mL), and the precipitated solid was collected by filtration and dried under reduced pressure to obtain a compound (1-2) (4.30 g, yield: 86%).
¹H-NMR (CDCl₃, 300 MHz): δ: 0.88 (6H, t), 1.15 to 1.52 (81H, m), 1.74 to 1.88 (4H, m), 4.77 to 4.88 (4H, m), 6.81 to 6.88 (2H, m), 6.94 (1H, d)

### <Synthesis of protective group-forming reagents (compound (1-3), compound (1-4), and compound (1-5))>

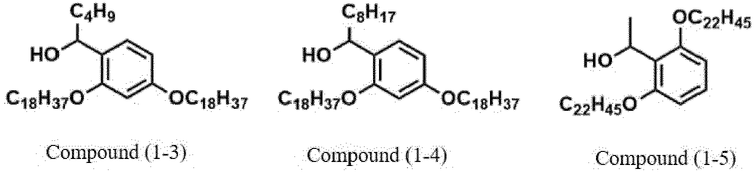

A compound (1-3) was obtained by the same method as in the compound (1-1).
¹H-NMR (CDCl₃, 300 MHz): δ = 0.86 to 0.91 (9H, m), 1.20 to 1.48 (64H, m), 1.74 to 1.81 (6H, m), 2.49 (1H, d), 3.91 to 3.97 (4H, m), 4.78 (1H, dt), 6.42 to 6.44 (2H, m), 7.15 (1H, d)

A compound (1-4) was obtained by the same method as in the compound (1-1).
¹H-NMR (CDCl₃, 300 MHz): δ = 0.83 to 0.91 (9H, m), 1.16 to 1.53 (72H, m), 1.71 to 1.89 (6H, m), 2.41 to 2.56 (1H, m), 3.88 to 3.99 (4H, m), 4.73 to 4.82 (1H, m), 6.40 to 6.46 (2H, m), 7.15 (1H, d)

A compound (1-5) was obtained by the same method as in the compound (1-2).
¹H-NMR (CDCl₃, 300 MHz): δ = 0.88 (6H, t), 1.19 to 1.53 (77H, m), 1.75 to 1.85 (4H, m), 3.49 (2H, d), 3.90 to 4.04 (4H, m), 4.08 (1H, d), 5.28 to 5.41 (1H, m), 6.52 (2H, d), 7.11 (1H, t)

### <Synthesis of protective group-forming reagent (compound (2-1))>

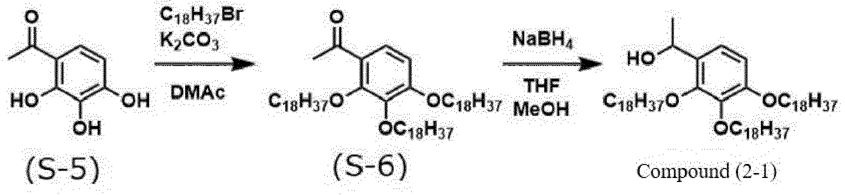

2,3,4-trihydroxyacetophenone (S-5) (10.0 g, 59.5 mmol), 1-bromodocosane (61.5 g, 184 mmol), potassium carbonate (37.0 g, 268 mmol), and N,N-dimethylacetamide (250 mL) were mixed and stirred at 110°C for 3 hours under a nitrogen atmosphere. After confirming the completion of the reaction, acetic acid and water were added thereto at 80°C. The mixture was allowed to cool to 50°C, and the precipitated solid was collected by filtration. After washing with water and methanol, an intermediate (S-6) (45.0 g, yield: 96%) was obtained by performing drying under reduced pressure.

Under a nitrogen atmosphere, the intermediate (S-6) (30.0 g, 35.6 mmol), tetrahydrofuran (150 mL), and methanol (15.0 mL) were mixed, sodium borohydride (1.35 g, 35.7 mmol) was added thereto at 40°C, and the mixture was stirred for 30 minutes. After completion of the reaction, acetone and a 20% aqueous solution of Rochelle salt were added dropwise thereto, and the mixture was allowed to cool to room temperature. The organic layer separated by an extraction operation was added dropwise to methanol (1.5 L), and the precipitated solid was collected by filtration and dried under reduced pressure to obtain a compound (2-1) (24.1 g, yield: 94%).

¹H-NMR (CDCl₃, 300 MHz): δ = 0.88 (9H, m), 1.14 to 1.59 (93H, m), 1.69 to 1.87 (6H, m), 2.53 (1H, d), 3.89 to 4.00 (4H, m), 4.02 to 4.16 (2H, m), 5.01 to 5.11 (1H, m), 6.62 (1H, d), 6.98 (1H, d)

### <Synthesis of protective group-forming reagents (compound (2-2), compound (2-3), compound (2-4), compound (2-5), and compound (2-6))>

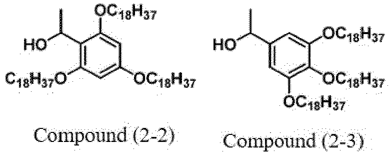

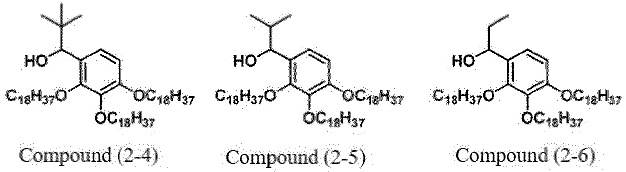

A compound (2-2) was obtained by the same method as in the compound (2-1).
¹H-NMR (CDCl₃, 300 MHz) δ = 0.88 (9H, t), 1.17 to 1.53 (99H, m), 1.69 to 1.86 (6H, m), 3.83 to 4.03 (7H, m), 5.20 to 5.32 (1H, m), 6.10 (2H, s)

A compound (2-3) was obtained by the same method as in the compound (1-1).
¹H-NMR (CDCl₃, 300 MHz) δ = 0.88 (9H, t), 1.18 to 1.53 (93H, m), 1.67 to 1.87 (7H, m), 3.89 to 4.05 (6H, m), 4.75 to 4.86 (1H, m), 6.56 (2H, s)

A compound (2-4) was obtained by the same method as in the compound (1-1), except that 2,3,4-trihydroxybenzaldehyde was used instead of (S-1) and tert-butylmagnesium bromide was used instead of methylmagnesium bromide.
¹H-NMR (CDCl₃, 300 MHz): δ = 6.98 (1H, d), 6.62 (1H, d), 4.69 (1H, s), 4.22 to 3.85 (6H, m), 1.89 to 1.67 (6H, m), 1.56 to 1.17 (90H, m), 0.92 (9H, s), 0.88 (9H, t)

A compound (2-5) was obtained by the same method as in the compound (1-1), except that 2,3,4-trihydroxybenzaldehyde was used instead of (S-1) and isopropylmagnesium bromide was used instead of methylmagnesium bromide.
¹H-NMR (CDCl₃, 300 MHz): δ = 6.90 (1H, d), 6.61 (1H, d), 4.45 (1H, d), 4.21 to 4.07 (2H, m), 4.03 to 3.89 (4H, m), 2.01 to 1.88 (1H, m), 1.86 to 1.68 (6H, m), 1.60 to 1.15 (90H, m), 1.04 (3H, d), 0.88 (9H, t), 0.79 (3H, d)

A compound (2-6) was obtained by the same method as in the compound (1-1), except that 2,3,4-trihydroxybenzaldehyde was used instead of (S-1) and ethylmagnesium bromide was used instead of methylmagnesium bromide.
¹H-NMR (CDCl₃, 300 MHz): δ = 6.95 (1H, d), 6.62 (1H, d), 4.75 (1H, s), 4.16 to 4.00 (2H, m), 3.94 (4H, t), 1.90 to 1.70 (8H, m), 1.55 to 1.18 (90H, m), 0.96 (3H, t), 0.88 (9H, t)

### <Synthesis of protective group-forming reagents (compound (2-7) and compound (2-8))>

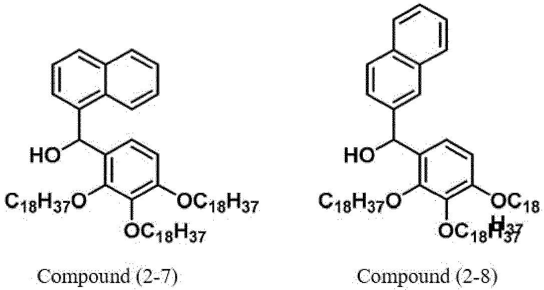

A compound (2-7) was obtained by the same method as in the compound (1-1), except that 2,3,4-trihydroxybenzaldehyde was used instead of (S-1), and 1-naphthyllithium (prepared from 1-bromonaphthalene and n-butyllithium) was used instead of methylmagnesium bromide.
¹H-NMR (CDCl₃, 300 MHz): δ = 7.98 (1H, d), 7.86 (1H, d), 7.80 (1H, d), 7.68 (1H, d), 7.53 to 7.39 (3H, m), 6.80 (1H, d), 6.59 (1H, d), 6.47 (1H, d), 4.10 to 3.94 (4H, m), 3.89 (2H, t), 2.88 (1H, d), 1.91 to 1.63 (8H, m), 1.60 to 0.96 (88H, m), 0.88 (9H, t)

A compound (2-8) was obtained by the same method as in the compound (1-1), except that 2,3,4-trihydroxybenzaldehyde was used instead of (S-1), and 2-naphthyllithium (prepared from 2-bromonaphthalene and n-butyllithium) was used instead of methylmagnesium bromide.
¹H-NMR (CDCl₃, 300 MHz): δ = 7.89 to 7.85 (1H, m), 7.84 to 7.75 (3H, m), 7.48 to 7.41 (3H, m), 6.84 (1H, d), 6.59 (1H, d), 6.10 (1H, s), 4.01 to 3.88 (4H, m), 3.78 to 3.72 (2H, m), 1.91 to 1.68 (8H, m), 1.65 to 1.11 (88H, m), 0.88 (9H, t)

### <Synthesis of protective group-forming reagents (compound (3-1), compound (3-2), and compound (3-3))>

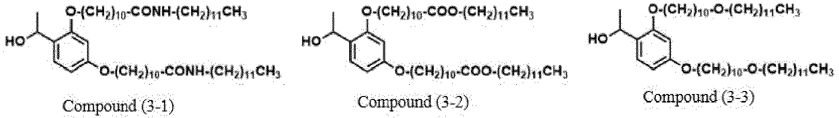

### <Synthesis of intermediate (3-2), intermediate (3-3), and intermediate (3-5)>

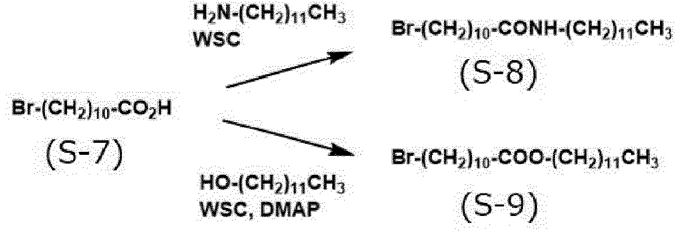

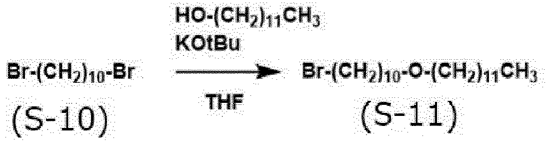

1-Ethyl-3-(3-dimethylaminopropyl)carbodiimide hydrochloride (WSC) (3.26 g, 17.0 mmol) was added to a mixed solution of 11-bromoundecanoic acid (S-7) (4.51 g, 17.0 mmol), dodecylamine (3.00 g, 16.2 mmol), and methylene chloride (32 mL) under a nitrogen atmosphere, and the mixture was stirred at room temperature for 1 hour. After confirming the completion of the reaction, ethyl acetate and saturated aqueous sodium bicarbonate were added thereto, the separated organic layer was dried over magnesium sulfate and then concentrated under reduced pressure. The residue was dissolved again in tetrahydrofuran (30 mL) and added dropwise to methanol (300 mL), and the precipitated solid was collected by filtration and dried under reduced pressure to obtain an intermediate (S-8) (4.47 g, yield: 64%).

1-Ethyl-3-(3-dimethylaminopropyl)carbodiimide hydrochloride (WSC) (1.24 g, 6.44 mmol) and N,N-dimethylaminopyridine (0.131 g, 1.07 mmol) was added to a mixed solution of 11-bromoundecanoic acid (S-7) (1.71 g, 6.44 mmol), 1-dodecanol (1.00 g, 5.37 mmol), and methylene chloride (11 mL), and the mixture was stirred at room temperature. After confirming the completion of the reaction, ethyl acetate and saturated aqueous sodium bicarbonate were added thereto, the separated organic layer was dried over magnesium sulfate and then concentrated under reduced pressure. The residue was dissolved again in ethyl acetate (5 mL) and added dropwise to methanol (50 mL), and the precipitated solid was collected by filtration and dried under reduced pressure to obtain an intermediate (S-9) (1.53 g, yield: 66%).

tert-butoxycalium (KOtBu) (6.02 g, 53.67 mmol)) was added to a mixed solution of 1,10-dibrodecane (S-10) (16.1 g, 53.7 mmol), 1-dodecanol (5.00 g, 26.8 mmol), and toluene (20 mL) at room temperature under a nitrogen atmosphere, and the mixture was stirred at 80°C for 3 hours. The reaction solution was allowed to cool to room temperature, a 10% ammonium chloride aqueous solution was added thereto, and the organic layer was separated and concentrated under reduced pressure. The residue was purified by silica gel column chromatography to obtain an intermediate (S-11) (4.7 g, yield: 41%).
¹H-NMR (CDCl₃, 300 MHz) δ = 0.88 (3H, t), 1.20 to 1.46 (50H, m), 1.51 to 1.61 (4H, m), 1.79 to 1.91 (2H, m), 3.35 to 3.45 (6H, m)

A compound (3-1) was obtained by the same method as in the compound (1-2) using the intermediate (S-8).
¹H-NMR (CDCl₃, 300 MHz): δ = 0.88 (6H, t), 1.20 to 1.51 (68H, m), 1.49 (3H, d), 1.72 to 1.84 (4H, m), 2.15 (4H, t), 2.76 (1H, br), 3.23 (4H, dt), 3.93 (2H, t), 3.97 (2H, t), 5.02 (1H, q), 5.45 (2H, br), 6.43 to 6.45 (2H, m), 7.19 (1H, d)

A compound (3-2) was obtained by the same method as in the compound (1-2) using the intermediate (S-9).
¹H-NMR (CDCl₃, 300 MHz): δ = 0.88 (6H, t), 1.21 to 1.51 (68H, m), 1.50 (3H, d), 1.72 to 1.83 (4H, m), 2.29 (4H, t), 2.66 (1H, br), 3.93 (2H, t), 3.97 (2H, t), 4.05 (4H, t), 5.03 (1H, q), 6.42 to 6.45 (2H, m), 7.19 (1H, d)

A compound (3-3) was obtained by the same method as in the compound (1-2) using the intermediate (S-11).
¹H-NMR (CDCl₃, 300 MHz): δ = 0.88 (6H, t), 1.16 to 1.67 (66H, m), 1.70 to 1.86 (4H, m), 2.68 (1H, d), 3.39 (8H, t), 3.93 (2H, t), 3.97 (2H, t), 4.98 to 5.10 (1H, m), 6.40 to 6.49 (2H, m), 7.19 (1H, d)

### <Synthesis of protective group-forming reagent (compound (4-1))>

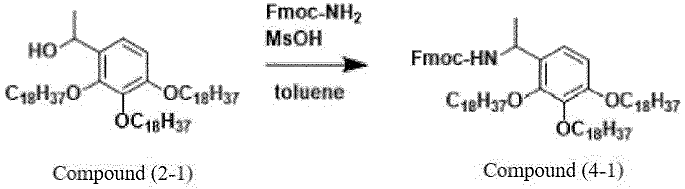

Under a nitrogen atmosphere, methane sulfonic acid (0.014 mL, 0.216 mmol) was added to a mixed solution of the compound (2-1) (1.00 g, 1.08 mmol), Fmoc-NH₂ (516 mg, 2.16 mmol), and toluene (10 mL) at 80°C, and the mixture was stirred for 30 minutes. The completion of the reaction was confirmed, and triethylamine (0.060 mL, 0.432 mmol) was added to the reaction solution, followed by allowing to stand to cool to room temperature. The reaction solution was added dropwise to methanol (100 mL), the precipitated solid was collected by filtration, dried under reduced pressure, and purified by silica gel column chromatography to obtain a compound (4-1) (0.98 g, yield: 77%).

¹H-NMR (CDCl₃, 400 MHz): δ = 0.88 (9H, t), 1.01 to 1.57 (91H, m), 1.68 to 1.90 (6H, m), 3.85 to 4.09 (6H, m), 4.09 to 4.24 (2H, m), 4.31 to 4.45 (2H, m), 4.90 to 5.05 (1H, m), 5.45 (1H, d), 6.59 (1H, d), 6.86 (1H, d), 6.86 (2H, t), 7.39 (2H, t), 7.58 (2H, d), 7.75 (2H, d)

### <Synthesis of protective group-forming reagents (compound (4-2), compound (4-3), and compound (4-4))>

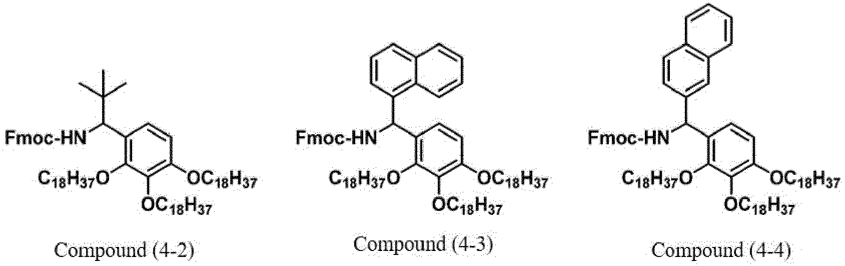

A compound (4-2) was synthesized by the same method as in the compound (4-1), except that the compound (2-4) was used instead of the compound (2-1).
¹H-NMR (CDCl₃, 300 MHz) δ = 7.81 to 7.70 (2H, m), 7.63 to 7.54 (2H, m), 7.45 to 7.22 (6H, m), 6.72 (1H, d), 6.58 (1H, d), 4.46 to 4.39 (2H, m), 4.38 to 4.27 (1H, m), 4.28 to 4.15 (2H, m), 4.09 to 3.85 (5H, m), 1.91 to 1.69 (6H, m), 1.58 to 1.14 (89H, m), 0.92 (9H, s), 0.88 (9H, t)

A compound (4-2) was synthesized by the same method as in the compound (4-1), except that the compound (2-7) was used instead of the compound (2-1).
¹H-NMR (CDCl₃, 300 MHz) δ = 8.19 to 6.42 (17H, m), 5.39 to 4.97 (1H, m), 4.51 to 4.17 (3H, m), 4.10 to 3.66 (7H, m), 1.92 to 1.66 (6H, m), 1.60 to 0.96 (90H, m), 0.88 (9H, t)

A compound (4-2) was synthesized by the same method as in the compound (4-1), except that the compound (2-8) was used instead of the compound (2-1).
¹H-NMR (CDCl₃, 300 MHz): δ = 7.88 to 7.19 (13H, m), 6.94 (1H, d), 6.64 (1H, d), 6.23 (1H, d), 6.01 (1H, d), 4.51 to 4.35 (2H, m), 4.33 to 4.22 (1H, m), 4.05 to 3.87 (6H, m), 3.47 to 3.34 (1H, m), 1.88 to 1.66 (6H, m), 1.60 to 0.96 (90H, m), 0.88 (9H, t)

### <Synthesis of protective group-forming reagent (compound (5-1))>

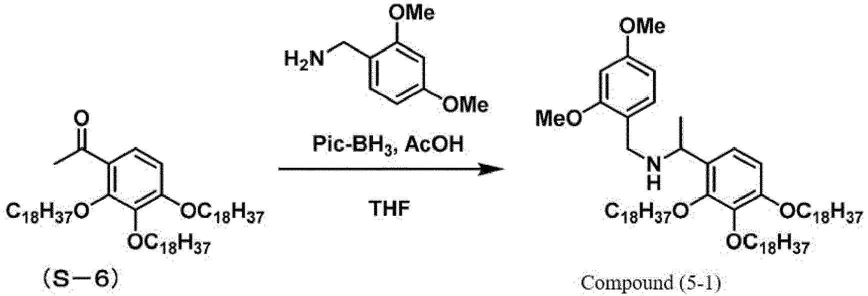

Under a nitrogen atmosphere, (S-6) (0.300 g, 0.314 mmol), 2,4-dimethoxybenzylamine (0.142 mL, 0.943 mmol), 2-picolineborane (67.2 mg, 0.63 mmol), acetic acid (0.054 mL), and tetrahydrofuran (2.0 mL) were mixed, and heated and refluxed for 5 hours. The mixture was allowed to return to room temperature after completion of the reaction, methanol (10 mL) was added thereto, and the precipitated solid was collected by filtration and then dried under reduced pressure. The resultant was purified by silica gel column chromatography to obtain a compound (5-1) (0.211 g, yield: 62%).
¹H-NMR (CDCl₃, 300 MHz): δ = 7.12 (2H, dd), 6.66 (1H, d), 6.45 to 6.36 (2H, m), 4.27 to 4.12 (1H, m), 4.05 to 3.87 (6H, m), 3.79 (6H, s), 3.72 to 3.61 (2H, m), 1.89 to 1.59 (8H, m), 1 .5 4 to 1.16 (92H, m), 0.88 (9H, t)

### <Synthesis of comparative protective group-forming reagent (comparative compound (1-1))>

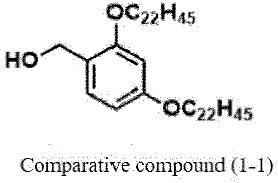

A comparative compound (1-1) was obtained by the same method as in the compound (2-1), except that the length of the alkyl group in the used raw material and the bromide was changed.

### (Example 1)

### <Synthesis of protected peptide compound (N-terminal and C-terminal protected peptide compound (1-1))>

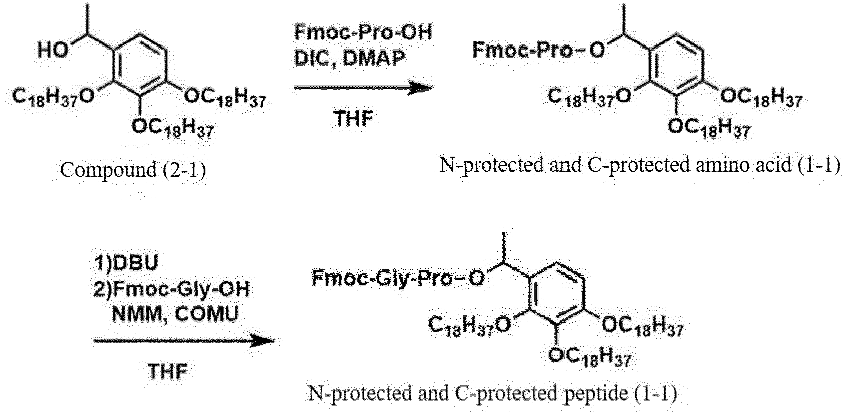

The compound (2-1) (300 mg, 0.32 mmol), N-[(9H-fluoren-9-ylmethoxy)carbonyl]-L-proline (164 mg, 0.49 mmol), and tetrahydrofuran (3.23 mL) were mixed at room temperature, and 4-dimethylaminopyridine (7.90 mg, 64.7 µmol) and diisopropylcarbodiimide (74.7 µL, 0.49 mmol) were added thereto. The reaction solution was stirred at 30°C for 1 hour, and then methanol (50 mL) was added thereto. The precipitated solid was collected by filtration and then dried under reduced pressure to obtain an N-protected and C-protected amino acid (1-1) (380 mg, 94%). Fmoc represents a 9-fluorenylmethoxycarbonyl group, and Pro represents a proline residue.

The N-protected and C-protected amino acid (1-1) (350 mg, 0.28 mmol) and tetrahydrofuran (2.81 mL) were mixed at room temperature, and diazabicycloundecene (50.4 µL, 0.34 mmol) was added thereto and stirred at room temperature for 1 hour. After confirming the completion of the reaction by high performance liquid chromatography (HPLC), N-methylmorpholine (63.3 µL, 0.58 mmol) and methanesulfonic acid (21.9 µL, 0.34 mmol) were successively added thereto, and then N-[(9H-fluoren-9-ylmethoxy)carbonyl]-L-glycine (100 mg, 0.34 mmol) and (1-cyano-2-ethoxy-2-oxoethylidenaminooxy)dimethylamino-morpholino-carbenium hexafluorophosphate (144 mg, 0.34 mmol) were added thereto. The reaction solution was stirred at 30°C for 30 minutes, and then acetonitrile (28.1 mL) was added to the reaction solution. The precipitated solid was collected by filtration, and then dried under reduced pressure to obtain an N-protected and C-protected peptide (1-1) (368 mg, 100%). Gly represents a glycine residue.

### (Comparative Example 1)

The comparative compound (1-1) was condensed with N-[(9H-fluoren-9-ylmethoxy)carbonyl]-L-proline (Fmoc-Pro-OH) and N-[(9H-fluoren-9-ylmethoxy)carbonyl]-L-glycine (Fmoc-Gly-OH) in the same manner as in the method for obtaining the N-protected and C-protected peptide (1-1), thereby synthesizing a corresponding N-protected and C-protected peptide.

### [Evaluation 1]

### <By-product rate of diketopiperazine (DKP)>

1.2 molar equivalent of 1,8-diazabicyclo[5.4.0]-7-undecene (DBU) was added to a tetrahydrofuran solution (0.38 mL) of the obtained N-protected and C-protected amino acid (50 mg), and the mixture was allowed to stand at 0°C for 1 hour. By NMR, amounts of the compound (2-1) and the comparative compound (1-1), generated as by-product of diketopiperazine, were determined. The results are shown in Table 1.

**[Table 1]**

| | Compound | By-product rate of DKP |
|---|---|---|
| Example 1 | Compound (2-1) | Not detected |
| Comparative Example 1 | Comparative compound (1-1) | 30% |

The same effect of suppressing the side reaction was also obtained in a case of using the compound (1-1), the compound (1-3), the compound (2-2), the compound (2-4), the compound (3-2), or the like. In a case where the substituted benzyl compound represented by Formula (1) of the present invention was used, the same effect of suppressing the side reaction could be obtained.

The substituted benzyl compound represented by Formula (1) of the present invention had excellent suppression property of side reaction (by-product of diketopiperazine) suppression as compared with the compound shown in Comparative Example 1, and thus has excellent yield of the peptide compound.

### <Synthesis of protective peptide (3-residue peptide: Fmoc-Arg(Pbf)-Cys(Trt)-Gly-O-protective group)>

Details of each abbreviation other than the above are shown below.
Gly: glycine residue
Cys(Trt): Trt-protected L-cysteine residue
Trt: trityl group
Arg(pbf): pbf-protected L-arginine residue
pbf: 2,2,4,6,7-pentamethyldihydrobenzofuran-5-sulfonyl group

### (Example 2: synthesis of Fmoc-Gly-O-TAG (1))

The compound (2-1) (0.835 g, 0.90 mmol) and Fmoc-Gly-OH (1.5 molar equivalent) were dissolved in tetrahydrofuran (9.0 mL), and 4-dimethylaminopyridine (0.2 molar equivalent) and diisopropylcarbodiimide (1.5 molar equivalent) were added thereto and stirred. After the condensation reaction was completed, methanol (MeOH, 90 mL) was added thereto and stirred, and the precipitate was collected by filtration and dried under reduced pressure to obtain Fmoc-Gly-O-TAG (1) (1.06 g, yield: 98%).

### (Example 3: synthesis of Fmoc-Cys(Mmt)-Gly-O-TAG (1))

Fmoc-Gly-O-TAG (1) (0.905 g, 0.750 mmol) was dissolved in tetrahydrofuran (15.0 mL), and diazabicycloundecene (DBU, 2.0 molar equivalent) was added thereto and stirred. After the deprotection reaction was completed, N-methylmorpholine (2.1 molar equivalent) and methanesulfonic acid (2.1 molar equivalent) were added thereto, and Fmoc-Cys(Mmt)-OH (1.2 molar equivalent) and (1-cyano-2-ethoxy-2-oxoethylidenaminooxy)dimethylamino-morpholino-carbenium hexafluorophosphate (COMU, 1.2 molar equivalent) were added thereto and stirred. After the condensation reaction was completed, acetonitrile (150 mL) was added thereto and stirred, and the precipitate was collected by filtration and dried under reduced pressure to obtain Fmoc-Cys(Mmt)-Gly-O-TAG (1) (1.11 g, yield: 96.6%).

### (Example 4: synthesis of Fmoc-Arg(Pbf)-Cys(Mmt)-Gly-O-TAG (1))

Fmoc-Cys(Mmt)-Gly-O-TAG (1) (0.854 g, 0.550 mmol) was dissolved in tetrahydrofuran (11 mL), and DBU (2.0 molar equivalent) was added thereto and stirred. After the deprotection reaction was completed, a chloroform solution including methanesulfonic acid (2.1 molar equivalent) and N-methylmorpholine (2.1 molar equivalent) was added thereto, and Fmoc-Arg(Pbf)-OH (1.25 molar equivalent) and COMU (1.25 molar equivalent) were added thereto and stirred. After the condensation reaction was completed, MeOH (110 mL) was added thereto and stirred, and the precipitate was collected by filtration and dried under reduced pressure to obtain Fmoc-Arg(Pbf)-Cys(Mmt)-Gly-O-TAG (1) (1.07 g, yield: 100%).

## Claims

1. A method for producing a peptide compound, comprising:
a step of using a substituted benzyl compound represented by Formula (1), 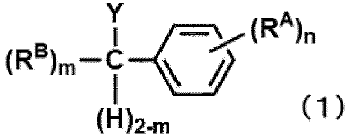
in Formula (1), Y represents -OH, -NHR, -SH, or -X, R represents a hydrogen atom, an alkyl group, an arylalkyl group, a heteroarylalkyl group, or a 9-fluorenylmethoxycarbonyl group, X represents Cl, Br, or I, m represents 1 or 2, n represents an integer of 1 to 5,
R^{B}'s each independently represent an aliphatic hydrocarbon group,
R^{A}'s each independently represent an aliphatic hydrocarbon group or an organic group having an aliphatic hydrocarbon group, where a number of carbon atoms in at least one aliphatic hydrocarbon group is 12 or more, and a benzene ring in Formula (1) may further have a substituent in addition to R^{A}.

2. The method for producing a peptide compound according to claim 1,
wherein the step of using the substituted benzyl compound represented by Formula (1) is a C-terminal protecting step of protecting a carboxy group or an amide group of an amino acid compound or a peptide compound with the substituted benzyl compound represented by Formula (1).

3. The method for producing a peptide compound according to claim 2,
wherein the amino acid compound or the peptide compound in the C-terminal protecting step is an N-terminal protected amino acid compound or an N-terminal protected peptide compound.

4. The method for producing a peptide compound according to claim 3, further comprising:
an N-terminal deprotecting step of deprotecting an N-terminal end of an N-terminal and C-terminal protected amino acid compound or an N-terminal and C-terminal protected peptide compound, which is obtained in the C-terminal protecting step; and
a peptide chain extending step of condensing the N-terminal end of a C-terminal protected amino acid compound or a C-terminal protected peptide compound, which is obtained in the N-terminal deprotecting step, with an N-terminal protected amino acid compound or an N-terminal protected peptide compound.

5. The method for producing a peptide compound according to claim 4, further comprising:
a precipitating step of precipitating an N-terminal and C-terminal protected peptide compound obtained in the peptide chain extending step.

6. The method for producing a peptide compound according to claim 5, further comprising, one or more times in the following order after the precipitating step:
a step of deprotecting an N-terminal end of the obtained N-terminal and C-terminal protected peptide compound;
a step of condensing the N-terminal end of the obtained C-terminal protected peptide compound with an N-terminal protected amino acid compound or an N-terminal protected peptide compound; and
a step of precipitating the obtained N-terminal and C-terminal protected peptide compound.

7. The method for producing a peptide compound according to any one of claims 1 to 6, further comprising:
a C-terminal deprotecting step of deprotecting a C-terminal protective group.

8. The method for producing a peptide compound according to any one of claims 1 to 7,
wherein a total number of carbon atoms in all aliphatic hydrocarbon groups included in all R^{A}'s is 36 to 80.

9. A protective group-forming reagent comprising:
a substituted benzyl compound represented by Formula (1), 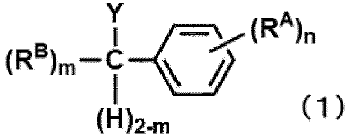
in Formula (1), Y represents -OH, -NHR, -SH, or -X, R represents a hydrogen atom, an alkyl group, an arylalkyl group, a heteroarylalkyl group, or a 9-fluorenylmethoxycarbonyl group, X represents Cl, Br, or I, m represents 1 or 2, n represents an integer of 1 to 5,
R^{B}'s each independently represent an aliphatic hydrocarbon group,
R^{A}'s each independently represent an aliphatic hydrocarbon group or an organic group having an aliphatic hydrocarbon group, where a number of carbon atoms in at least one aliphatic hydrocarbon group is 12 or more, and a benzene ring in Formula (1) may further have a substituent in addition to R^{A}.

10. The protective group-forming reagent according to claim 9,
wherein the protective group-forming reagent is a reagent for forming a protective group of a carboxy group or an amide group.

11. The protective group-forming reagent according to claim 9 or 10,
wherein the protective group-forming reagent is a C-terminal protective group-forming reagent of an amino acid compound or a peptide compound.

12. A substituted benzyl compound represented by Formula (1), 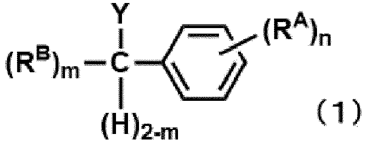
in Formula (1), Y represents -OH, -NHR, -SH, or -X, R represents a hydrogen atom, an alkyl group, an arylalkyl group, a heteroarylalkyl group, or a 9-fluorenylmethoxycarbonyl group, X represents Cl, Br, or I, m represents 1 or 2, n represents an integer of 1 to 5,
R^{B}'s each independently represent an aliphatic hydrocarbon group,
R^{A}'s each independently represent an aliphatic hydrocarbon group or an organic group having an aliphatic hydrocarbon group, where a number of carbon atoms in at least one aliphatic hydrocarbon group is 12 or more, and a benzene ring in Formula (1) may further have a substituent in addition to R^{A}.

13. A method for producing a peptide compound, comprising:
a step of using a substituted benzyl compound represented by Formula (1), 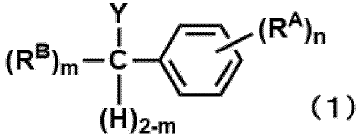
in Formula (1), Y represents -OH, -NHR, -SH, or -X, R represents a hydrogen atom, an alkyl group, an arylalkyl group, a heteroarylalkyl group, or a 9-fluorenylmethoxycarbonyl group, X represents Cl, Br, or I, m represents 1 or 2, n represents an integer of 1 to 5,
R^{B}'s each independently represent an aromatic group (excluding a phenyl group),
R^{A}'s each independently represent an aliphatic hydrocarbon group or an organic group having an aliphatic hydrocarbon group, where a number of carbon atoms in at least one aliphatic hydrocarbon group is 12 or more, and a benzene ring in Formula (1) may further have a substituent in addition to R^{A}.

14. The method for producing a peptide compound according to claim 13, wherein R^{B} represents a naphthalene group.
